(19) Europäisches Patentamt
European Patent Office
Office européen des brevets

(11) EP 1 909 775 B1

(12) **EUROPEAN PATENT SPECIFICATION**

(45) Date of publication and mention of the grant of the patent:
**16.05.2012 Bulletin 2012/20**

(21) Application number: **06788612.7**

(22) Date of filing: **28.07.2006**

(51) Int Cl.:
***A61K 31/10*** (2006.01) ***A61K 31/192*** (2006.01)
***A61K 31/724*** (2006.01) ***A61P 39/00*** (2006.01)
***A61K 47/16*** (2006.01) ***A61K 47/32*** (2006.01)
***A61K 47/34*** (2006.01) ***A61K 47/40*** (2006.01)

(86) International application number:
**PCT/US2006/029109**

(87) International publication number:
**WO 2007/016201 (08.02.2007 Gazette 2007/06)**

(54) **FORMULATION OF RADIOPROTECTIVE ALPHA, BETA UNSATURATED ARYL SULFONES**

FORMULIERUNG STRAHLENSCHÜTZENDER, ALPHA, BETA UNGESÄTTIGTER ARYLSULFONE

FORMULATION D'ARYL SULFONES NON SATURES ALPHA, BETA RADIOPROTECTEURS

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.07.2005 US 704236 P**

(43) Date of publication of application:
**16.04.2008 Bulletin 2008/16**

(73) Proprietor: **Onconova Therapeutics, Inc.**
**Newtown, PA 18940 (US)**

(72) Inventors:
- **MANIAR, Manoj**
**Fremont, California 94539 (US)**
- **BELL, Stanley, C.**
**Narbeth, Pennsylvania 19072 (US)**

(74) Representative: **Howard, Paul Nicholas et al**
**Carpmaels & Ransford**
**One Southampton Row**
**London**
**WC1B 5HA (GB)**

(56) References cited:
**WO-A-2006/010152 US-A- 4 834 985**
**US-A1- 2003 060 505 US-A1- 2003 060 505**
**US-B2- 6 656 973**

- **STRICKLEY R G: "SOLUBILIZING EXCIPIENTS IN ORAL AND INJECTABLE FORMULATIONS" PHARMACEUTICAL RESEARCH, KLUWER ACADEMIC PUBLISHERS, NEW YORK, NY, US, vol. 21, no. 2, 1 February 2004 (2004-02-01), pages 201-230, XP009035738 ISSN: 0724-8741**
- **ALFIERI A A ET AL: "Radiation damage protection by the benzyl styrl sulfone analog, Ex-Rad" INTERNATIONAL JOURNAL OF RADIATION ONCOLOGY BIOLOGY PHYSICS, vol. 60, no. 1, Suppl. S, 2004, pages S367-S368, XP002549965 & 46TH ANNUAL MEETING OF THE AMERICAN-SOCIETY-FOR-THERAPEUTIC-RADIOLOGY AND-ONCOLOGY; ATLANTA, GA, USA; OCTOBER 03 -07, 2004 ISSN: 0360-3016**
- **DASH S K ET AL.: "Preformulation development of a parenteral formulation for ON 01210.Na, a radioprotectant" AAPS ANNUAL MEETING AND EXPOSITION, [Online] 5 November 2005 (2005-11-05), - 10 November 2005 (2005-11-10) pages 1-1, XP002549966 Retrieved from the Internet: URL:http:// abstracts.aapspharmaceutica.com /ExpoAAPS05/CC/forms/attendee/index.aspx?content=sessionInfo&sessionId=577> [retrieved on 2009-10-05]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 909 775 B1

## Description

### FIELD OF THE INVENTION

The invention relates to formulations for pharmacological delivery of cytoprotective agents, e.g., at least one α, β unsaturated aryl sulfone, for the protection and/or treatment of cells and tissues from/of the toxicity of ionizing radiation.

### BACKGROUND OF THE INVENTION

While anti-radiation suits or other protective gear may be effective at reducing radiation exposure, such gear is expensive, unwieldy, and generally not available to public. Moreover, radioprotective gear will not protect normal tissue adjacent a tumor from stray radiation exposure during radiotherapy. Pharmaceutical radioprotectants offer a cost-efficient, effective and easily available alternative to radioprotective gear. However, previous attempts at radioprotection of normal cells with pharmaceutical compositions have not been entirely successful. For example, cytokines directed at mobilizing the peripheral blood progenitor cells confer a myeloprotective effect when given prior to radiation (Neta et al., Semin. Radiat. Oncol. 6:306-320, 1996), but do not confer systemic protection. Other chemical radioprotectors administered alone or in combination with biologic response modifiers have shown minor protective effects in mice, but application of these compounds to large mammals was less successful, and it was questioned whether chemical radioprotection was of any value (Maisin, J.R., Bacq and Alexander Award Lecture. "Chemical radioprotection: past, present, and future prospects", Int J. Radiat Biol. 73:443-50, 1998). Pharmaceutical radiation sensitizers, which are known to preferentially enhance the effects of radiation in cancerous tissues, are clearly unsuited for the general systemic protection of normal tissues from exposure to ionizing radiation.

US2003/0060505 describes a method for protecting cells and tissues from ionizing radiation with α,β-unsaturated aryl sulfones. Alfieri et al. (Proceedings of the 46th Annual ASTRO Meeting, International Journal of Radiation Oncology Biology Physics, 60(1)Suppl. S, S367-S368, 2004) describes radiation damage protection by a benzyl styrl sulfone analog, Ex-Rad. US-B2-6656973 describes (E)-4-carboxystyryl-4-chlorobenzyl sulfone.

What is needed, therefore, is a practical means to protect subjects from the toxicity of radiation, wherein the subjects are either scheduled to incur or are at risk for incurring, or have incurred, exposure to ionizing radiation.

### SUMMARY OF THE INVENTION

The present invention is directed to pharmaceutical compositions for administration for reducing toxic effects of ionizing radiation in a subject, comprising between about 20 mg/ml to about 60 mg/ml of the sodium salt of (E)-4-carboxystyryl-4-chlorobenzylsulfone (ON.1210.Na) and at least one component selected from the group consisting of a) at least one chemically modified cyclodextrin selected from the group consisting of 2-hydroxypropyl-beta-cyclodextrin, 2-hydroxypropyl-gamma-cyclodextrin, and hydroxyethyl-beta-cyclodextrin in an amount between about 20% and about 60% w/v, b) a water soluble polymer selected from the group consisting of povidone in an amount between about 0.5% and about 20% w/v and PEG in an amount between about 25% and about 90% w/v, and c) DMA in an amount between about 10% and about 50% w/v, wherein the composition has a pH within the range of about 7.5 to about 9.2.

Preferred example pharmaceutical compositions of the present invention comprise between about 30 mg/ml to about 50 mg/ml of the compound ON.1210.Na ((E)-4-Carboxystyryl-4-chlorobenzylsulfone, sodium salt ($C_{16}H_{12}ClNaO_4S$)).

### BRIEF DESCRIPTION OF THE FIGURES

FIG. 1A and 1B show the effect of 5 Gy and 10 Gy of ionizing radiation, respectively, on the viability of DU145 prostate tumor cells in the presence or absence of (E)-4-fluorostyryl-4-chlorobenzylsulfone.

FIG. 2A and 2B show the effect of 5 Gy and 10 Gy of ionizing radiation, respectively, on the viability of DU145 prostate tumor cells in the presence or absence of (E)-4-carboxystyryl-4-chlorobenzylsulfone.

FIG. 3A and 3B show the effect of 10 Gy ionizing radiation on the viability of DU145 prostate tumor cells treated post-irradiation, respectively, with (E)-4-fluorostyryl-4-chlorobenzylsulfone and (E)-4-carboxystyryl-4-chlorobenzylsulfone.

FIG. 4 is a plot of average body weight (grams) vs. time(days) for C57B6/J mice given 4 mg/kg (E)-4-fluorostyryl-4-chlorobenzylsulfone every other day for 18 days.

FIG. 5 is a Kaplan Meyer survival plot of C57B6/J mice pre-treated with (E)-4-carboxystyryl-4-chlorobenzylsulfone

at 18 and 6 hrs before receiving 8 Gy of ionizing radiation.

FIG. 6 is a Kaplan Meyer survival plot of C57B6/J mice treated with (E)-4-carboxystyryl-4-chlorobenzylsulfone after receiving 8 Gy of ionizing radiation.

FIG. 7 shows the structure of the sodium salt of 4-chlorobenzyl-4-carboxystyryl sulfone (ON.1210.Na).

FIG. 8 shows a UV Scan of ON.1210.Na in water.

FIG. 9 shows representative chromatograms of ON.1210.Na: (a) the mobile phase, (b) standard solution (250 μg/mL), (c) ON.1210.Na in 0.1N NaOH before autoclaving, and (d) ON.1210.Na in 0.1N NaOH after autoclaving.

FIG. 10A illustrates a mobile phase without ON.1210.Na (Blank). FIG. 10B shows a representative chromatogram ofON.1210.Na using an isocratic system.

FIG. 11 shows pH solubility profiles ofON.1210.Na at ambient temperature.

FIG. 12 illustrates degradation of ON.1210.Na as a function of pH at 75˚C.

FIG. 13 shows pseudo first-order rate constant for the degradation of ON.1210.Na as a function of pH at 75˚C.

FIG. 14 shows powder XRPD pattern of ON.1210 free acid.

FIG. 15 shows powder XRPD pattern ofON.1210.Na.

FIG. 16 shows powder XRPD pattern of ON.1210.Na precipitated from an aquesous solution.

## DETAILED DESCRIPTION OF THE INVENTION

Unless defined otherwise, all technical and scientific terms used herein have the same meaning as is commonly understood by one of skill in the art to which this invention belongs. The entireties of the disclosures of U.S. Patent Nos. 6,656,973 and 6,667,346 are particularly referenced.

The major biological effects of radiation exposure are the destruction of bone marrow cells, gastrointestinal (GI) damage, lung pneumonitis, and central nervous system (CNS) damage. The long-term effects of radiation exposure include an increase in cancer rates. It has been estimated that the exposure of 100 rems (roentgen equivalent man: a measurement used to quantify the amount of radiation that would produce harmful biological effects) would produce ARS symptoms. Exposure levels above 300 rems would result in the death of approximately 50% of the exposed population.

The α, β-unsaturated aryl sulfones, in particular benzyl styryl sulfones, provide significant and selective systemic protection of normal cells from radiation-induced damage in animals. When used in radiotherapy techniques, these compounds also exhibit independent toxicity to cancer cells. Compositions and formulations of α,β unsaturated aryl sulfones described herein protect normal cells and tissues from the effects of acute and chronic exposure to ionizing radiation. The α,β unsaturated aryl sulfones are also operationally cytotoxic in tumor cells. Compositions described herein are intended for prophylactic use, for example, to enhance survival in personnel who are in imminent danger of exposure to life-threatening levels of x-ray or gamma radiation, and/or to enhance survival in personnel who have just received life-threatening levels of xray or gamma radiation. In animal efficacy studies, pre-treatment with ON.01210.Na, for example, by the intravenous, sub-cutaneous or intraperitoneal route resulted in protection of mice from a lethal dose of ionizing radiation.

Subjects may be exposed to ionizing radiation when undergoing therapeutic irradiation for the treatment of proliferative disorders. Such disorders include cancerous and non-cancer proliferative disorders. Formulations described herein are effective in protecting normal cells during therapeutic irradiation of a broad range of tumor types, including but not limited to the following: breast, prostate, ovarian, lung, colorectal, brain (i.e., glioma) and renal. The compositions are also effective against leukemic cells, for example. The compositions are useful in protecting normal cells during therapeutic irradiation of abnormal tissues in non-cancer proliferative disorders, including but not limited to hemangiomatosis in new born, secondary progressive multiple sclerosis, chronic progressive myelodegenerative disease, neurofibromatosis, ganglioneuromatosis, keloid formation, Paget's Disease of the bone, fibrocystic disease of the breast, Peronies and Duputren's fibrosis, restenosis and cirrhosis.

Therapeutic ionizing radiation may be administered to a subject on any schedule and in any dose consistent with the prescribed course of treatment, as long as the α,β unsaturated aryl sulfone radioprotectant compound is administered prior to the radiation. The course of treatment differs from subject to subject, and those of ordinary skill in the art can readily determine the appropriate dose and schedule of therapeutic radiation in a given clinical situation. Compositions of α,β unsaturated aryl sulfones described herein should be administered far enough in advance of the therapeutic radiation such that the compound is able to reach the normal cells of the subject in sufficient concentration to exert a radioprotective effect on the normal cells. At least one α,β unsaturated aryl sulfone may be administered as much as about 24 hours, preferably no more than about 18 hours, prior to administration of the radiation. In one embodiment, an α,β unsaturated aryl sulfone formulation is administered at least about 6 - 12 hours before administration of the therapeutic radiation. Most preferably, the α,β unsaturated aryl sulfone is administered once at about 18 hours and again at about 6 hours before the radiation exposure. One or more α,β unsaturated aryl sulfones may be administered simultaneously, or different α,β unsaturated aryl sulfones may be administered at different times during the treatment.

Preferably, an about 24 hour period separates administration of α,β unsaturated aryl sulfone and the therapeutic radiation. More preferably, the administration of α,β unsaturated aryl sulfone and the therapeutic radiation is separated by about 6 to 18 hours. This strategy will yield significant reduction in radiation-induced side effects without affecting the anticancer activity of the therapeutic radiation.

An acute dose of ionizing radiation which may cause remediable radiation damage includes a localized or whole body dose, for example, between about 10,000 millirem (0.1 Gy) and about 1,000,000 millirem (10 Gy) in 24 hours or less, preferably between about 25,000 millirem (0.25 Gy) and about 200,000 (2 Gy) in 24 hours or less, and more preferably between about 100,000 millirem (1 Gy) and about 150,000 millirem (1.5 Gy) in 24 hours or less.

A chronic dose of ionizing radiation which may cause remediable radiation damage includes a whole body dose of about 100 millirem (.001 Gy) to about 10,000 millirem (0.1 Gy), preferably a dose between about 1000 millirem (.01 Gy) and about 5000 millirem (.05 Gy) over a period greater than 24 hours, or a localized dose of 15,000 millirem (0.15 Gy) to 50,000 millirem (0.5 Gy) over a period greater than 24 hours.

In the event of a terrorist attack releasing lethal amounts of radiation, radioprotective compositions described herein should provide protection when administered just *after,* e.g., up to about four hours after, exposure.

I. STRUCTURAL CLASSES

The compositions of the present invention comprise the sodium salt of (E)-4-carboxystyryl-4-chlorobenrylsulfone (ON.1210.Na), which is an α,β-unsaturated aryl sulfone. By "α,β unsaturated aryl sulfone" as used herein is meant a chemical compound containing one or more α,β unsaturated aryl sulfone groups:

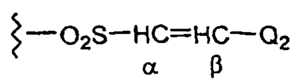

wherein $Q_2$ is substituted or unsubstituted aryl, and the hydrogen atoms attached to the α and β carbons are optionally replaced by other chemical groups.

By "substituted" means that an atom or group of atoms has replaced hydrogen as the substituent attached to a ring atom. The degree of substitution in a ring system may be mono-, di-, tri- or higher substitution.

The term "aryl" employed alone or in combination with other terms, means, unless otherwise stated, a carbocyclic aromatic system containing one or more rings (typically one, two or three rings) wherein such rings may be attached together in a pendent manner or may be fused. Examples include phenyl; anthracyl; and naphthyl, particularly 1-naphthyl and 2-naphthyl. The aforementioned listing of aryl moieties is intended to be representative, not limiting. It is understood that the term "aryl" is not limited to ring systems with six members.

The term "heteroaryl" by itself or as part of another substituent means, unless otherwise stated, an unsubstituted or substituted, stable, mono- or multicyclic heterocyclic aromatic ring system which consists of carbon atoms and from one to four heteroatoms selected from the group consisting ofN, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom which affords a stable structure.

Examples of such heteroaryls include benzimidazolyl, particularly 2-benzimidazolyl; benzofuryl, particularly 3-, 4-, 5-, 6- and 7-benzofuryl; 2-benzothiazolyl and 5-benzothiazolyl; benzothienyl, particularly 3-, 4-, 5-, 6-, and 7-benzothienyl; 4-(2-benzyloxazolyl); furyl, particularly 2- and 3-furyl; isoquinolyl, particularly 1- and 5-isoquinolyl; isoxazolyl, particularly 3-, 4- and 5-isoxazolyl; imidazolyl, particularly 2-, -4 and 5-imidazolyl; indolyl, particularly 3-, 4-, 5-, 6- and 7-indolyl; oxazolyl, particularly 2-, 4- and 5-oxazolyl; purinyl; pyrrolyl, particularly 2-pyrrolyl, 3-pyrrolyl; pyrazolyl, particularly 3- and 5-pyrazolyl; pyrazinyl, particularly 2-pyrazinyl; pyridazinyl, particularly 3- and 4-pyridazinyl; pyridyl, particularly 2-, 3- and 4-pyridyl; pyrimidinyl, particularly 2- and 4-pyrimidyl; quinoxalinyl, particularly 2- and 5-quinoxalinyl; quinolinyl, particularly 2- and 3-quinolinyl; 5-tetrazolyl; 2-thiazolyl; particularly 2-thiazolyl, 4-thiazolyl and 5-thiazolyl; thienyl, particularly 2- and 3-thienyl; and 3-(1,2,4-triazolyl). The aforementioned listing of heteroaryl moieties is intended to be representative, not limiting.

In one class, the α,β unsaturated aryl sulfone group is a styryl sulfone group:

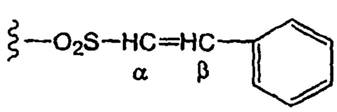

wherein the hydrogen atoms attached to the α and β carbons are optionally replaced by other chemical groups, and the phenyl ring is optionally substituted.

By "styryl sulfone" or "styryl sulfone compound" or "styryl sulfone therapeutic" as used herein is meant a chemical compound containing one or more such styryl sulfone groups.

The α,β unsaturated aryl sulfone radioprotective compounds are characterized by cis-trans isomerism resulting from the presence of a double bond. Stearic relations around a double bond are designated as "Z" or "E". Both configurations are included in the scope of "α,β unsaturated aryl sulfone":

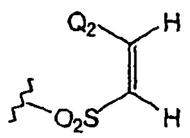
Z configuration

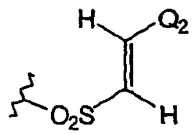
E configuration

In one class, the α,β unsaturated aryl sulfone compound is a compound of the formula I:

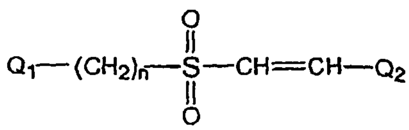
I

wherein:

n is one or zero;
$Q_1$ and $Q_2$ are, same or different, are substituted or unsubstituted aryl, or substituted or unsubstituted heteroaryl.

In one class, n in formula I is one, that is, the compounds comprise α,β unsaturated benzylsulfones, e.g. styryl benzylsulfones.

In one class according to formula I, $Q_1$ and/or $Q_2$ are selected from substituted and unsubstituted heteroaryl; for example, (E)-3-furanethenyl-2,4-dichlorobenzylsulfone.

In another class according to formula 1, $Q_1$ and $Q_2$ are selected from substituted and unsubstituted phenyl.

In one class compounds where $Q_1$ and $Q_2$ are selected from substituted and unsubstituted phenyl comprise compounds of the formula II:

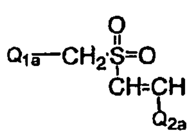
II

wherein:

$Q_{1a}$ and $Q_{2a}$ are independently selected from the group consisting of phenyl and mono-, di-, tri-, tetra- and penta-substituted phenyl where the substituents, which may be the same or different, are independently selected from the group consisting of hydrogen, halogen, C1-C8 alkyl, C1-C8 alkoxy, nitro, cyano, carboxy, hydroxy, phosphonato, amino, sulfamyl, acetoxy, dimethylamino(C2-C6 alkoxy), C1-C6 trifluoroalkoxy and trifluoromethyl.

In one class, compounds of formula II are at least di-substituted on at least one ring, that is, at least two substituents on at least one ring are other than hydrogen. In another embodiment, compounds of formula II are at least trisubstituted on at least one ring, that is, at least three substituents on at least one ring are other than hydrogen.

In one class, the radioprotective compound has the formula III:

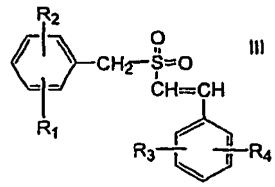

III

wherein $R_1$, $R_2$, $R_3$ and $R_4$ are independently selected from the group consisting of hydrogen, halogen, C1-C8 alkyl, C1-C8 alkoxy, nitro, cyano, carboxy, hydroxy phosphonato, amino, sulfamyl, acetoxy, dimethylamino(C2-C6 alkoxy), C1-C6 trifluoroalkoxy and trifluoromethyl.

In one class, the radioprotective compound is according to formula III, and $R_1$ and $R_2$ are independently selected from the group consisting of hydrogen, halogen, cyano, and trifluoromethyl; and $R_3$ and $R_4$ are independently selected from the group consisting of hydrogen and halogen.

In one sub class of formula III, the radioprotective α,β unsaturated aryl sulfone compound is a compound of the formula IIIa, wherein $R_2$ and $R_4$ are other than hydrogen:

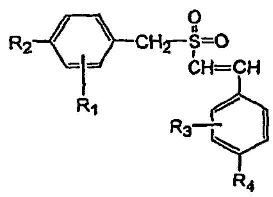

IIIa

Compounds according to formula IIIa having the E-configuration include, but are not limited to, (E)-4-fluorostyryl-4-chlorobenzylsulfone; (E)-4-chlorostyryl-4-chlorobenzylsulfone; (E)-2-chloro-4-fluorostyryl-4-chlorobenzylsulfone; (E)-4-carboxystyryl-4-chlorobenzyl sulfone; (E)-4-fluorostyryl-2,4-dichlorobenzylsulfone; (E)-4-fluorostyryl-4-bromobenzylsulfone; (E)-4-chlorostyryl-4-bromobenzylsulfone; (E)-4-bromostyryl-4-chlorobenzylsulfone; (E)-4-fluorostyryl-4-trifluoromethylbenzylsulfone; (E)-4-fluorostyryl-3,4-dichlorobenzylsulfone; (E)-4-fluorostyryl-4-cyanobenzylsulfone; (E)-2,4-dichloro-4-chlorobenzylsulfone; (E)-4-fluorostyryl-4-chlorophenylsulfone and (E)-4-chlorostyryl-2,4-dichlorobenzylsulfone.

In another class, compounds of formula IIIa have the Z configuration wherein $R_1$ and $R_3$ are hydrogen, and $R_2$ and $R_4$ are selected from the group consisting of 4-halogen. Such compounds include, for example, (Z)-4-chlorostyryl-4-chlorobenzylsulfone; (Z)-4-chlorostyryl-4-fluorobenzylsulfone; (Z)-4-fluorostyryl-4-chlorobenzylsulfone; (Z)-4-bromostyryl-4-chlorobenzylsulfone; and (Z)-4-bromostyryl-4-fluorobenzylsulfone.

In another class, the radioprotective α,β unsaturated aryl sulfone compound is a compound of the formula IV:

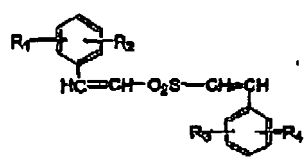

IV

wherein

$R_1$, $R_2$, $R_3$, and $R_4$ are independently selected from the group consisting of hydrogen, halogen, C1-8 alkyl, C1-8 alkoxy, nitro, cyano, carboxy, hydroxy, and trifluromethyl.

In One class, $R_1$ in formula IV is selected from the group consisting of hydrogen, chlorine, fluorine and bromine; and $R_2$, $R_3$ and $R_4$ are hydrogen In one class the compound of formula IV is (Z)-styryl-(E)-2-methoxy-4-ethoxystyrylsulfone.

In another class, the radioprotective α,β unsaturated aryl sulfone compound is a compound of the formula V:

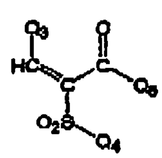

V

wherein

$Q_3$, $Q_4$ aud $Q_5$ are independently selected from the group consisting of phenyl and mono-, di-, tri-, tetra- and penta-substituted phenyl where the substituents, which may be the same or different are independently selected from the groups cousisting of halogen, C1-C8 alkyl, C1-C8 alkoxy, nitro, cyano, carboxy, hydroxy, phosphonato, amino, sulfamyl, acetoxy, dimethylamino(C2-C6 alkoxy C1-C6 triftuoroalkoxy and trifluoromethyl.

In one sub-class of formula V, the radioprotective α,β unsaturated aryl sulfone compound is a compound of the formula Va:

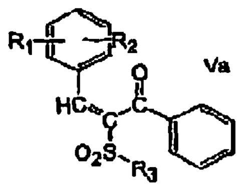

Va

wherein

$R_1$ and $R_2$ are independently selected from the group consisting of hydrogen, halogen, C1-C8 alkyl, C1-8 alkoxy, nitro, cyano, carboxyl, hydroxyl, and trifluoromethyl; and

$R_3$ is selected from the group consisting of unsubstituted phenyl, monosubstituted phenyl and di-substituted phenyl, the substituents on the phenyl ring being independently selected from the group consisting of halogen and C1-8 alkyl.

Preferably, $R_1$ in formula Va is selected from the group consisting of fluorine and bromine; $R_2$ is hydrogen; and $R_3$ is selected from the group consisting of 2-chlorophenyl, 4-chlorophenyl, 4-fluorophenyl, and 2-nitrophenyl.

A radioprotective styryl sulfone according to formula Va is the compound wherein $R_1$ is fluorine, $R_2$ is hydrogen and $R_3$ is phenyl, that is, the compound 2-(phenylsulfonyl)-1-phenyl-3-(4-fluorophenyl)-2-propen-1-one.

By "dimethylamino(C2-C6 alkoxy)" is meant $(CH_3)_2N(CH_2)_nO$- wherein n is from 2 to 6. Preferably, n is 2 or 3. Most preferably, n is 2, that is, the group is the dimethylaminoethoxy group, that is, $(CH_3)_2NCH_2CH_2O$-.

By "phosphonato" is meant the group $-PO(OH)_2$.

By "sulfamyl" is meant the group $-SO_2NH_2$.

Where a substituent on an aryl nucleus is an alkoxy group, the carbon chain may be branched or straight, with straight being preferred. Preferably, the alkoxy groups comprise C1-C6 alkoxy, more preferably C1-C4 alkoxy, most preferably methoxy.

(E)-α,β unsaturated aryl sulfones may be prepared by Knoevenagel condensation of aromatic aldehydes with benzylsulfonyl acetic acids or arylsulfonyl acetic acids. The procedure is described by Reddy et al., Acta. Chim. Hung. 115:269-71 (1984); Reddy et al., Sulfur Letters 13:83-90 (1991); Reddy et al., Synthesis No. 4, 322-23 (1984); and Reddy et al., Sulfur Letters 7:43-48 (1987), the entire disclosures of which are incorporated herein by reference. See, particularly, the entire disclosures of U.S. Patent Nos. 6,656,973 and 6,667,346.

The α,β unsaturated aryl sulfones may take the form or pharmaceutically acceptable salts. The term "pharmaceutically acceptable salts" embraces salts commonly used to form alkali metal salts and to form addition salts of free acids or free bases. The nature of the salt is not critical, provided that it is pharmaceutically-acceptable.

**Table 1: Compounds**

| | Compound |
|---|---|
| 1 | (E)-styryl phenyl sulfone |
| 2 | (E)-4-chlorostyryl phenyl sulfone |
| 3 | (E)-2,4-dichlorostyryl phenyl sulfone |
| 4 | (E)-4-bromostyryl phenyl sulfone |
| 5 | (E)-4-chlorostyryl 4-chlorophenyl sulfone |
| 6 | (B)-4-methylstyryl 4-chlorophenyl sulfone |
| 7 | (E)-4-methoxystyryl 4-chlorophenyl sulfone |
| 8 | (E)-4-bromostyryl 4-chlorophenyl sulfone |
| 9 | (E)-2-chlorostyryl benzyl sulfone |
| 10 | (E)-4-chlorostyryl benzyl sulfone |

(continued)

| | Compound |
|---|---|
| 11 | (E)-4-fluorostyryl 4-chlorobenzyl sulfone |
| 12 | (E)-4-chlorostyryl 4-chlorobenzyl sulfone |
| 13 | (E)-4-fluorostyryl 4-fluorobenzyl sulfone |
| 14 | (E)-2,4-difluorostyryl 4-fluorobenzyl sulfone |
| 15 | (E)-4-fluorostyryl 4-bromobenzyl sulfone |
| 16 | (E)-4-bromostyryl 4-bromobenzyl sulfone |
| 17 | (E)-bromostyryl 4-fluorobenzyl sulfone |
| 18 | (E)-4-chlorostyryl 4-bromobenzyl sulfone |
| 19 | (E)-4-bromostyryl 4-chlorobenzyl sulfone |
| 20 | (E)-4-fluorostyryl-4-trifluoromethylbenzylsulfone |
| 21 | (E)-4-chlorostyryl-4-trifluoromethylbenzylsulfone |
| 22 | (E)-4-bromostyryl-4-trifluoromethylbenzylsulfone |
| 23 | (E)-4-fluorostyryl-2,4-dichlorobenzylsulfone |
| 24 | (E)-4-chlorostyryl-2,4-dichlorobenzylsulfone |
| 25 | (E)-4-fluorostyryl-3,4-dichlorobenzylsulfone |
| 26 | (E)-4-chlorostyryl-3,4-dichlorobenzylsulfone |
| 27 | (E)-4-bromostyryl-3,4-dichlorobenzylsulfone |
| 28 | (E)-4-bromostyryl-4-nitrobenzylsulfone |
| 29 | (E)-4-fluorostyryl-4-cyanobenzylsulfone |
| 30 | (E)-4-chlorostyryl-4-cyanobenzylsulfone |
| 31 | (E)-4-bromostyryl-4-cyanobenzylsulfone |
| 32 | (E)-3,4-difluorostyryl-4-chlorobenzylsulfone |
| 33 | (E)-3-chloro-4-fluorostyryl-4-chlorobenzylsulfone |
| 34 | (E)-2-chloro-4-fluorostyryl-4-chlorobenzylsulfone |
| 35 | (E)-2,4-dichlorostyryl-4-chlorobenzylsulfone |
| 36 | (E)-3,4-dichlorostyryl-4-chlorobenzylsulfone |
| 37 | (E)-2,3-dichlorostyryl-4-chlorobenzylsulfone |
| 38 | (Z)-styryl benzylsulfone |
| 39 | (Z)-styryl 4-chlorobenzylsulfone |
| 40 | (Z)-styryl 2-chlorobenzylsulfone |
| 41 | (Z)-styryl 4-fluorobenzylsulfone |
| 42 | (Z)-4-chlorostyryl benzylsulfone |
| 43 | (Z)-4-chlorostyryl 4-chlorobenzylsulfone |
| 44 | (Z)-4-chlorostyryl 2-chlorobenzylsulfide |
| 45 | (Z)-4-chlorostyryl 4-fluorobenzylsulfone |
| 46 | (Z)-4-fluorostyryl benzylsulfone |
| 47 | (Z)-4-fluorostyryl 4-chlorobenzylsulfone |
| 48 | (Z)-4-fluorostyryl 2-chlorobenzylsulfone |

(continued)

| | Compound |
|---|---|
| 49 | (Z)-4-fluorostyryl 4-fluorobenzylsulfone |
| 50 | (Z)-4-bromostyryl benzylsulfone |
| 51 | (Z)-4-bromostyryl 4-chlorobenzylsulfone |
| 52 | (Z)-4-bromostyryl 2-chlorobenzylsulfone |
| 53 | (Z)-4-bromostyryl 4-fluorobenzylsulfone |
| 54 | (Z)-4-methylstyryl benzylsulfone |
| 55 | (Z)-4-methylstyryl 4-chlorobenzylsulfone |
| 56 | (Z)-4-methylstyryl 2-chlorobenzylsulfone |
| 57 | (Z)-4-methylstyryl 4-fluorobenzylsulfone |
| 58 | (E)-2-nitrostyryl-4-fluorobenzylsulfone |
| 59 | (E)-3-nitrostyryl-4-fluorobenzylsulfone |
| 60 | (E)-4-nitrostyryl-4-fluorobenzylsulfone |
| 61 | (E)-2-trifluoromethylstyryl-4-fluorobenzylsulfone |
| 62 | (E)-3-trifluoromethylstyryl-4-fluorobenzylsulfone |
| 63 | (E)-4-trifluoromethylstyryl-4-fluorobenzylsulfone |
| 64 | (E)-2-trifluoromethyl-4-fluorostyryl-4-fluorobenzylsulfone |
| 65 | (E)-2-nitrostyryl-4-chlorobenzylsulfone |
| 66 | (E)-3-nitrostyryl-4-chlorobenzylsulfone |
| 67 | (E)-4-nitrostyryl-4-chlorobenzylsulfone |
| 68 | (E)-2-trifluoromethylstyryl-4-chlorobenzylsulfone |
| 69 | (E)-3-trifluoromethylstyryl-4-chlorobenzylsulfone |
| 70 | (E)-4-trifluoromethylstyryl-4-chlorobenzylsulfone |
| 71 | (E)-2-trifluoromethyl-4-fluorostyryl-4-chlorobenzylsulfone |
| 72 | (E)-3-methyl-4-fluorostyryl-4-chlorobenzylsulfone |
| 73 | (E)-2-nitrostyryl-2,4-dichlorobenzylsulfone |
| 74 | (E)-2-trifluoromethyl-4-fluorostyryl-2,4-dichlorobenzylsulfone |
| 75 | (E)-2-nitrostyryl-4-bromobenzylsulfone |
| 76 | (E)-3-nitrostyryl-4-bromobenzylsulfone |
| 77 | (E)-4-nitrostyryl-4-bromobenzylsulfone |
| 78 | (E)-2-trifluoromethylstyryl-4-bromobenzylsulfone |
| 79 | (E)-3-trifluoromethylstyryl-4-fluorobenzylsulfone |
| 80 | (E)-4-trifluoromethylstyryl-4-bromobenzylsulfone |
| 81 | (E)-2-nitrostyryl-4-cyanobenzylsulfone |
| 82 | (E)-3-nitrostyryl-4-cyanobenzylsulfone |
| 83 | (E)-4-nitrostyryl-4-cyanobenzylsulfone |
| 84 | (E)-4-fluorostyryl-4-methylbenzylsulfone |
| 85 | (E)-4-bromostyryl-4-methylbenzylsulfone |
| 86 | (E)-2-nitrostyryl-4-methylbenzylsulfone |

(continued)

| | Compound |
|---|---|
| 87 | (E)-3-nitrostyryl-4-methylbenzylsulfone |
| 88 | (E)-4-nitrostyryl-4-methylbenzylsulfone |
| 89 | (E)-4-fluorostyryl- 4-methoxybenzylsulfone |
| 90 | (E)-4-chlorostyryl-4-methoxybenzylsulfone |
| 91 | (E)-4-bromostyryl-4-methoxybenzylsulfone |
| 92 | (E)-2-nitrostyryl-4-methoxybenzylsulfone |
| 93 | (E)-3-nitrostyryl-4-methoxybenzylsulfone |
| 94 | (E)-4-nitrostyryl-4-methoxybenzylsulfone |
| 95 | (E)-4-chlorostyryl-4-nitrobenzylsulfo-ne |
| 96 | (E)-4-fluorostyryl-4-nitrobenzylsulfone |
| 97 | (E)-2,3,4,5,6-pentafluorostyryl-4-fluorobenzylsulfone |
| 98 | (E)-2,3,4,5,6-pentafluorostyryl-4-chlorobenzylsulfone |
| 99 | (E)-2,3,4,5,6-pentafluorostyryl-4-bromobenzylsulfone |
| 100 | (E)-4-fluorostyryl-2,3,4,5,6-pentafluorobenzylsulfone |
| 101 | (E)-4-chlorostyryl-2,3,4,5,6-pentafluorobenzylsulfone |
| 102 | (E)-4-bromostyryl-2,3,4,5,6-pentafluorobenzylsulfone |
| 103 | (E)-2,3,4,5,6-pentafluorostyryl-3,4-dichlorobenzylsulfone |
| 104 | (E)-2,3,4,5,6-pentafluorostyryl-2,3,4,5,6-pentafluorobenzylsulfone |
| 105 | (E)-2,3,4,5,6-pentafluorostyryl-4-iodobenzylsulfone |
| 106 | (E)-2-hydroxy-3,5-dinitrostyryl-4-fluorobenzylsulfone |
| 107 | (E)-2-hydroxy-3,5-dinitrostyryl-4-bromobenzylsulfone |
| 108 | (E)-2-hydroxy-3,5-dinitrostyryl-4-chlorobenzylsulfone |
| 109 | (E)-2-hydroxy-3,5-dinitrostyryl-2,4-dicchlorobenzylsulfone |
| 110. | (E)-2,4,6-trimethoxystyryl-4-methoxybenzylsulfone |
| 111 | (E)-3-methyl-2,4-dimethoxystyryl-4-methoxybenzylsulfone |
| 112 | (E)-3,4,5-trimethoxystyryl-4-methoxybenzylsulfone |
| 113 | (E)-3,4,5-trimethoxystyryl-2-nitro-4,5-dimethoxybenzylsulfone |
| 114 | (E)-2,4,6-trimethoxystyryl-2-nitro-4,5-dimethoxybenzylsulfone |
| 115 | (E)-3-methyl-2,4-dimethoxystyryl-2-nitro-4,5-dimethoxybenzylsulfone |
| 116 | (E)-2,3,4-trifluorostyryl-4-fluorobenzylsulfone |
| 117 | (E)-2,3,4-trifluorostyryl-4-chlorobenzylsulfone |
| 118 | (E)-2,6-dimethoxy-4-hydroxystyryl-4-methoxybenzylsulfone |
| 119 | (E)-2,3,5,6-tetrafluorostyryl-4-methoxybenzylsulfone |
| 120 | (E)-2,4,5-trimethoxystyryl-4-methoxybenzylsulfone |
| 121 | (E)-2,3,4-trimethoxystyryl-4-methoxybenzylsulfone |
| 122 | (E)-3-nitro-4-hydroxy-5-methoxystyryl-4-methoxybenzylsulfone |
| 123 | (E)-3,4-dimethoxy-6-nitrostyryl-4-methoxybenzylsulfone |
| 124 | (E)-3,4-dimethoxy-5-iodostyryl-4-methoxybenzylsulfone |

(continued)

| | Compound |
|---|---|
| 125 | (E)-2,6-dimethoxy-4-fluorostyryl-4-methoxybenzylsulfone |
| 126 | (E)-2-hydroxy-4,6-dimethoxystyryl-4-methoxybenzylsulfone |
| 127 | (E)-2,4,6-trimethylstyryl-4-methoxybenzylsulfone |
| 128 | (E)-2,4,6-trimethoxystyryl-4-chlorobenzylsulfone |
| 129 | (E)-2,6-dimethoxy-4-fluorostyryl-4-chlorobenzylsulfone |
| 130 | (E)-2-hydroxy-4,6-dimethoxystyryl-4-chlorobenzylsulfone |
| 131 | (E)-2,4,6-trimethoxystyryl-4-bromobenzylsulfone |
| 132 | (E)-2,6-dimethoxy-4-fluorostyryl-4-bromobenzylsulfone |
| 133 | (E)-2,4,6-trimethoxystyryl-2,3,4-trimethoxybenzylsulfone |
| 134 | (E)-2,6-dimethoxystyryl-2,3,4-trimethoxybenzylsulfone |
| 135 | (E)-2,4,6-trimethoxystyryl-,3,4,5-trimethoxybenzylsulfone |
| 136 | (E)-2,6-dimethoxystyryl-3,4,5-trimethoxybenzylsulfone |
| 137 | (E)-4-fluorostyryl-2,3,4-trimethoxybenzylsulfone |

In one class, α,β unsaturated aryl sulfone formula 1a:

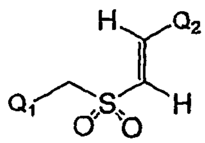

Ia

wherein $Q_1$ and $Q_2$ are, same or different, are substituted or unsubstituted aryl, or substituted or unsubstituted heteroaryl illustrated by Table 2 below.

**Table 2: Formula 1a Wherein $Q_1$ And $Q_2$ Are:**

| | $Q_1$ | $Q_2$ |
|---|---|---|
| 138 | 4-fluorophenyl | 2-pyridyl |
| 139 | 4-fluorophenyl | 3-pyridyl |
| 140 | 4-fluorophenyl | 4-pyridyl |
| 141 | 4-chlorophenyl | 2-pyridyl |
| 142 | 4-chlorophenyl | 3-pyridyl |
| 143 | 4-chlorophenyl | 4-pyridyl |
| 144 | 4-bromophenyl | 2-pyridyl |
| 145 | 4-bromophenyl | 3-pyridyl |
| 146 | 4-bromophenyl | 4-pyridyl |
| 147 | 4-fluorophenyl | 2-thienyl |
| 148 | 4-chlorophenyl | 2-thienyl |
| 149 | 4-bromophenyl | 2-thienyl |
| 150 | 4-fluorophenyl | 4-bromo-2-thienyl |
| 151 | 4-chlorophenyl | 4-bromo-2-thienyl |
| 152 | 4-bromophenyl | 4-bromo-2-thienyl |

(continued)

| | $Q_1$ | $Q_2$ |
|---|---|---|
| 153 | 4-fluorophenyl | 5-bromo-2-thienyl |
| 154 | 4-chlorophenyl | 5-bromo-2-thienyl |
| 155 | 4-bromophenyl | 5-bromo-2-thienyl |
| 156 | 4-fluorophenyl | 2-thienyl-1,1-dioxide |
| 157 | 4-chlorophenyl | 2-thienyl-1,1-dioxide |
| 158 | 4-bromophenyl | 2-thienyl-1,1-dioxide |
| 159 | 4-fluorophenyl | 3-thienyl |
| 160 | 4-chlorophenyl | 3-thienyl |
| 161 | 4-bromophenyl | 3-thienyl |
| 162 | 4-iodophenyl | 3-thienyl |
| 163 | 4-methylphenyl | 3-thienyl |
| 164 | 4-methoxyphenyl | 3-thienyl |
| 165 | 4-trifluoro-methylphenyl | 3-thienyl |
| 166 | 2,4-dichlorophenyl | 3-thienyl |
| 167 | 3,4-dichlorophenyl | 3-thienyl |
| 168 | 4-cyanophenyl | 3-thienyl |
| 169 | 4-nitrophenyl | 3-thienyl |
| 170 | 4-fluorophenyl | 3-thienyl-1,1-dioxide |
| 171 | 4-chlorophenyl | 3-thienyl-1,1-dioxide |
| 172 | 4-bromophenyl | 3-thienyl-1,1-dioxide |
| 173 | 4-methoxyphenyl | 3-thienyl-1,1-dioxide |
| 174 | 2,4-dichlorophenyl | 3-thienyl-1,1-dioxide |
| 175 | 4-fluorophenyl | 2-furyl |
| 176 | 4-chlorophenyl | 2-furyl |
| 177 | 4-bromophenyl | 2-furyl |
| 178 | 4-fluorophenyl | 3-furyl |
| 179 | 4-chlorophenyl | 3-furyl |
| 180 | 4-bromophenyl | 3-furyl |
| 181 | 4-iodophenyl | 3-furyl |
| 182 | 4-methylphenyl | 3-furyl |
| 183 | 4-methoxyphenyl | 3-furyl |
| 184 | 4-trifluoro-methylphenyl | 3-furyl |
| 185 | 2,4-dichlorophenyl | 3-furyl |
| 186 | 3,4-dichlorophenyl | 3-furyl |
| 187 | 4-cyanophenyl | 3-furyl |
| 188 | 4-nitrophenyl | 3-furyl |
| 189 | 4-chlorophenyl | 2-thiazolyl |
| 190 | 4-chlorophenyl | 2-pyrrolyl |

(continued)

| | $Q_1$ | $Q_2$ |
|---|---|---|
| 191 | 4-bromophenyl | 2-pyrrolyl |
| 192 | 4-chlorophenyl | 2-nitro-4-thienyl |
| 193 | 4-iodophenyl | 2-nitro-4-thienyl |
| 194 | 2,4-dichlorophenyl | 2-nitro-4-thienyl |
| 195 | 4-methoxyphenyl | 2-nitro-4-thienyl |
| 196 | 4-fluorophenyl | 1-naphthyl |
| 197 | 4-fluorophenyl | 2-naphthyl |
| 198 | 4-chlorophenyl | 1-naphthyl |
| 199 | 4-chlorophenyl | 2-naphthyl |
| 200 | 4-bromophenyl | 1-naphthyl |
| 201 | 4-bromophenyl | 2-naphthyl |
| 202 | 1-naphthyl | 4-fluorophenyl |
| 203 | 1-naphthyl | 4-chlorophenyl |
| 204 | 1-naphthyl | 4-bromophenyl |
| 205 | 1-naphthyl | 2-nitrophenyl |
| 206 | 1-naphthyl | 3-nitrophenyl |
| 207 | 1-naphthyl | 4-nitrophenyl |
| 208 | 4-fluorophenyl | 9-anthryl |
| 209 | 4-chlorophenyl | 9-anthryl |
| 210 | 4-bromophenyl | 9-anthryl |

**Table 3**

| | Compound |
|---|---|
| 211 | (E)-styryl phenyl sulfone |
| 212 | (E)-4-chlorostyryl phenyl sulfone |
| 213 | (E)-2,4-dichlorostyryl phenyl sulfone |
| 214 | (E)-4-bromostyryl phenyl sulfone |
| 215 | (E)-4-chlorostyryl 4-chlorophenyl sulfone |
| 216 | (E)-4-methylstyryl 4-chlorophenyl sulfone |
| 217 | (E)-4-methoxystyryl 4-chlorophenyl sulfone |
| 218 | (E)-4-bromostyryl 4-chlorophenyl sulfone |
| 219 | (E)-2-chlorostyryl benzyl sulfone |

Polymorphs of all compounds disclosed and contemplated herein are intended to be within the scope of the disclosure.

## II. EXAMPLE SPECIES

An exemplary species of a radioprotective $\alpha,\beta$ unsaturated arylsulfone is **ON.1210.Na.** ON.1210.Na is a derivative of chlorobenzylsulfone. This and related compounds are described herein as exhibiting valuable prophylactic properties which mitigate the effects of accidental and intentional exposure to life-threatening levels of irradiation. Hence,

a systematic development of this and related compounds is described with the objective of developing a shelf stable formulations.

Table 4 describes the general physical properties of ON.1210.Na. The example compound is a sodium salt of (E)-4-Carboxystyryl-4-chlorobenzylsulfone.

**Table 4: Physical Properties of ON.1210.Na**

| | |
|---|---|
| Chemical Structure | 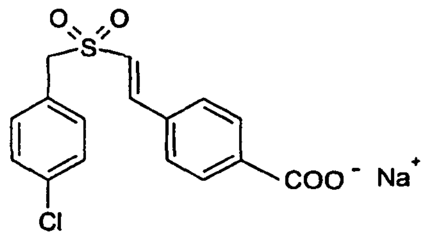 |
| Chemical Name | (E)-4-Carboxystyryl-4-chlorobenzylsulfone, Sodium Salt |
| Empirical Formula | $C_{16}H_{12}ClNaO_4S$ |
| Molecular Weight | 358.79 |
| Physical Nature | White crystalline flakes |
| Melting Point | 354-356˚C |
| Solubility | Soluble in water at 8-10 mg/ml |

The compound ON 01210.Na appears to form at least one polymorph. X-ray diffraction pattern, for example, of precipitated ON 01210.Na is different from that of the originally synthesized compound. Polymorphs of ON 01210.Na are intended to be within the scope of the claims appended hereto.

Treatment of normal human fibroblasts with ON 01210.Na, for example, prior to exposure to cytotoxic levels of ionizing radiation provides dose-dependent radio-protection.

The physio-chemical properties ofON.1210.Na, as an example drug substance, was determined in order to evaluate appropriate formulation approaches for development of a safe, reliable and effective parenteral formulation of these compounds. This includes microscopic studies, partition coefficient, pKa, pH solubility studies, pH stability studies under accelerated conditions, solid state characterization of the drug substance, and solid state stability of drug substance under accelerated conditions. *See*, section IV, *infra.* This example compound has a low octanol:water partition coefficient (1.28-2.87). The equilibrium solubility of the drug at pH 4.0, 5.0, 6.0, 7.4, 8.0, 9.0 was 0.000154, 0.0379, 0.715, 11.09, 16.81, 23.3 mg/mL, respectively. The pKa calculated from pH-solubility studies was 2.85± 0.6. The pH-stability profile of the drug indicated better stability at neutral and biological pH but degradation was rapid under acidic conditions. The degradation followed a pseudo-first-order rate. The accelerated solid-state stability study of the bulk drug substance showed no evidence of degradation. This drug is quite stable in an aqueous environment at biological pH. Therefore, it can be formulated as a shelf stable parenteral formulation. The aqueous solubility of the drug as the free acid is low and can be significantly enhanced by increase in pH, co-solvents and complexation.

## III. FORMULATIONS OF RADIOPROTECTANT α, β UNSATURATED ARYL SULFONES

Although compositions described and contempated herein are not so limited, due to the oral, for example, bioavailability of the compounds upon administration, a preferred route of administration of the compositions described herein include, for example, parenteral administration. Parenteral administration includes intravenous, intramuscular, intraarterial, intraperitoneal, intravaginal, intravesical (e.g., into the bladder), intradermal, topical or subcutaneous administration. The α,β unsaturated aryl sulfone may be administered in the form of a pharmaceutical composition comprising one or more α,β unsaturated aryl sulfones in combination with a pharmaceutically acceptable carrier. The α,β unsaturated aryl sulfone in such formulations may comprise from 0.1 to 99.99 weight percent. By "pharmaceutically acceptable carrier" is meant any carrier, diluent or excipient which is compatible with the other ingredients of the formulation and is not deleterious to the subject.

The specific dose and schedule of α,β unsaturated aryl sulfone to obtain the radioprotective benefit will, of course, be determined by the particular circumstances of the individual patient including, the size, weight, age and sex of the patient, the nature and stage of the disease being treated, the aggressiveness of the disease, and the route of administration, and the specific toxicity of the radiation. For example, a daily dosage of from about 0.01 to about 150

mg/kg/day may be utilized, more preferably from about 0.05 to about 50 mg/kg/day. Particularly preferred are doses from about 1.0 to about 10.0 mg/kg/day, for example, a dose of about 7.0 mg/kg/day. The dose may be given over multiple administrations, for example, two administrations of 3.5 mg/kg. Higher or lower doses are also contemplated.

For parenteral administration, the α,β unsaturated aryl sulfones may be mixed with a suitable carrier or diluent such as water, an oil, saline solution, aqueous dextrose (glucose) and related sugar solutions, cyclodextrins or a glycol such as propylene glycol or polyethylene glycol as described infra. Solutions for parenteral administration preferably contain a pharmaceutically acceptable, water soluble salt of the α,β unsaturated aryl sulfone. Stabilizing agents, antioxidizing agents, chelating agents, and preservatives, for example, may also be added. Suitable antioxidizing agents include sulfite, ascorbic acid, citric acid and its salts, and sodium EDTA as a chelator, for example. Suitable preservatives include benzalkonium chloride, methyl- or propyl-paraben, and chlorbutanol.

**Shelf-stable pharmaceutical compositions which comprise effective amount of at least one radioprotective α,β unsaturated aryl sulfone compound are described herein.** The term "effective amount" of at least one radioprotective α,β unsaturated aryl sulfone compound, for example, as used herein refers to an amount of compound, after dilution as described infra, that is effective to mitigate, reduce or eliminate toxicity associated with radiation in normal cells of the subject and/or to impart a direct cytotoxic effect to abnormally proliferating cells in the subject. As used with respect to bone marrow purging, "effective amount of the radioprotective α,β unsaturated aryl sulfone compound" refers to an amount of at least one α,β unsaturated aryl sulfone effective to reduce or eliminate the toxicity associated with radiation in bone marrow removed from a subject and/or to impart a direct cytotoxic effect to malignant cells in the bone marrow removed from the subject. Each α,β unsaturated arylsulfone is administered, for example, in a concentration of about 0.25 micromolar to about 100 micromolar; preferably, from about 1.0 to about 50 micromolar; more preferably from about 2.0 to about 25 micromolar. Particularly preferred concentrations for administration are, for example, about 0.5, 1.0 and 2.5 micromolar and about 5, 10 and 20 micromolar. Higher or lower concentrations may also be used depending upon factors well known in the art.

The formulations described and claimed herein are generally intended for dilution and subsequent parenteral administration. Compositions of the present invention are generally formulated with active ingredient, e.g., one or more compounds described herein, in a concentrated form for storage and handling prior to dilution with suitable parenteral diluent prior to infusion. Preferred shelf-stable compositions of the present invention, for dilution prior to administration, generally comprise between about 10 mg/ml to about 90 mg/ml of at least one α,β unsaturated aryl sulfone. Preferred compositions of the present invention comprise between about 20 mg/ml to about 80 mg/ml of at least one α,β unsaturated aryl sulfone. Example compositions of the present invention comprise between about 30 mg/ml to about 50 mg/ml (e.g., about 40mg/ml) of at least one α,β unsaturated aryl sulfone (namely, (E)-4-carboxysyryl-4-chlorobenzylsulfone sodium salt (ON.1210.Na)); wherein the composition exhibits a pH within the range of about 7.2 to about 9.2.

A single dosage is generally within the range of about 1 ml to about 5 ml of any of the compositions described herein. Individual 3 ml dosages of compositions described herein are contemplated, for example. The dosages may be packaged, for example, in 5 ml vials. Compositions of the present invention may, for example, be diluted with about 7 parts diluent (7:1) prior to administration. However, the dilution factor and the diluent employed depend on the concentration of drug in the formulation. Compositions of the present invention, however, may be diluted with anywhere, for example, within the range of about 2 volumes of suitable parenteral diluent prior to infusion to about 12 volumes of suitable parenteral diluent, prior to infusion. The final diluted product for parenteral administration of compositions of the present invention should have a pH within the range of about 6.5 to about 10.0. Preferably the final diluted product for parenteral administration should have a pH within the range of about 7.0 to about 9.5 A *diluted* product pH of about 7.0 to about 8.0 is preferred.

The osmolarity of the diluted formulation for administration should be approximately within the range of about 200 to about 400 mOsm/kg. Preferred osmolarity of the diluted formulation for administration should be approximately within the range of about 270 to about 330 mOsm/kg. A preferred osmolarity of the diluted formulation for administration should be approximately 300 mOsm/kg.

Compositions described herein, unless specified otherwise, are preferrably aqueous-based; however, ethanol, for example, is also a preferred base-component of the formulations described herein.

DMA (N,N-dimethylacetamide) is an example preferred component of formulations described herein. Compositions otherwise described herein, further discussed *infra*, are particularly contemplated that comprise between about 15% and about 40% by weight of DMA. Composition for parenteral administration comprising an effective amount of a compound described herein and about 20% to about 35% by weight of DMA are example preferred embodiments. An example composition of the present invention comprises an effective amount of at least one radioprotective α, β unsaturated aryl sulfone compound in a formulation wherein components DMA:WATER;ETHANOL exist in an approximate 1:1:1 ratio, for example. This example ratio, however, can be significantly modified by those of ordinary skill in the art with simple and straightforward experimentation so long as the solubility of the compound remains to provide an effective amount for drug delivery. Another example composition of the present invention comprises an effective amount of at least one radioprotective α, β unsaturated aryl sulfone compound in a formulation wherein components DMA:WATER:

and PEG400, for example, (at least one water soluble polymer (WSP)) exist in an approximate 1:2:2 ratio, for example. However in this example (DMA:WATER:WSP) the 'water soluble polymer' component can be much greater, e.g., as described *infra*, in many embodiments at least about 40% by weight of at least one water soluble polymer. The language herein, generally, also concerning preferred drug concentrations and pH values of the compositions, similarly applies to DMA formulations contemplated herein.

A composition for parenteral administration comprising an effective amount of a compound described herein and at least about 0.5% by weight of at least one water soluble polymer is provided. Formulations of the present invention have a pH within a range of about 7.5 to about 9.2. High pH, e.g., about 8.5, is preferred. Compositions are preferred that comprise between about 5% and about 90% of at least one water soluble polymer, e.g. at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, and at least about 85%. The term "water soluble polymer", as used herein, includes but is not limited to water soluble polymer excipients known in the art including polyethylene glycol (PEG), polypropylene glycol, poly-oxyethylene, poly-oxyethylene-poly-oxypropylene copolymer, polyglycerol, polyvinylalcohol, polyvinylpyrrolidone (PVP) (Povidone (soluble homopolymer of N-vinyl-2-pyrrolidone)), polyvinylpyridine N-oxide, copolymer of vinylpyridine N-oxide and vinylpyridine, and the like, as well as derivatives thereof, and combinations thereof.

Poly-oxyethylene and/or poly-oxyethylene-poly-oxypropylene copolymers are example water-soluble polymers for use in formulations of the present invention. Poloxamer 407 (e.g., Pluronic F 127, Lμtrol® micro 127), for example, and/or Poloxamer 188 (e.g., Pluronic F 68, Lμtrol® micro 68) are poly-oxyethylene-poly-oxypropylene copolymers that can be used in combination in formulations of the present invention. BASF Corporation, Mount Olive, NJ.

Polyethylene glycols (PEGs) are preferred water soluble polymers. Low molecular weight liquid polyethylene glycols, for example, PEG 300, PEG 400, PEG 600, and PEG 800, are preferred water soluble polymers that can be used independently or in combination with each other, for example, in formulations of the present invention. Particularly preferred are PEG 300, PEG 400, and PEG 600. Lutrol® E 300, Lutrol® E 400 and Lutrol® E 600, for example, are commercially available from BASF Corporation, Mount Olive, NJ. PEG 400 (Polyethylene glycol 400, Macrogol 400, PEG 400, Poly(oxy-1,2-ethanediyl),alpha-hydro-omega-hydroxy- (CAS No: 25322-68-3)), e.g., Lutrol® E 400, is preferred. Example water soluble polymers selected from the group consisting essentially of PEG 300, PEG 400, PEG 600, and PEG 800 are preferred. Although not specifically listed here PEG products substantially the same, otherwise within this characteristic range of PEG entities, may be employed in compositions of the present invention.

Low molecular weight povidone grades (CAS No: 9003-39-8), Kollidon® 12 PF and Kollidon® 17 PF, for example, form a soluble complex between the compounds and povidone. Both products are available as pyrogen-free powders, suitable for use in injectables, from BASF Corporation, Mount Olive, NJ. Kollidon® 12 PF, Kollidon® 17 PF, Kollidon® 25, Kollidon® 30, and Kollidon® 90 F are preferred examples of povidone. Compositions of the present invention are exemplified herein that comprise Povidone K 90, for example, at concentrations of about 1% to about 5% in water. These compositions are further contemplated, for example, that comprise Povidone K 90, for example, at concentrations of about 0.5% to about 10% in water. Low molecular weight Povidones, for example, may be employed at much higher concentrations in compositions of the present invention, e.g., between about 5% by weight and about 20% by weight, depending on the viscosity of the formulation.

Chemically Modified Cyclodextrins are preferred components of compositions described herein. Cyclodextrin drug compositions described herein are easily diluted, for example, eliminating the need for elaborate mixing procedures. Chemically modified cyclodextrin, hydroxypropyl-b-cyclodextrin, for example, formulations are preferred for parenteral administration due to the ability to of the cyclodextrins to solubilize and stabilize the compounds described herein as well as the superior safety profile exhibited by the chemically modified cyclodextrins. The use of cyclodextrins referred to herein can reduce dosing volume and *in situ* irritation resulting from high pH, for example, other solvents, or any direct chemical irritancy due to the compounds otherwise described herein.

Many different chemical moieties may be introduced into the Cyclodextrin molecule by reaction with the hydroxyl groups lining the upper and lower ridges of the toroid; for example, hydroxypropyl, carboxymethyl, and acetyl. Since each Cyclodextrin hydroxyl groups differs in its chemical reactivity, reaction processes produces an amorphous mixture of thousands of positional and optical isomers. Examples of chemically modified cyclodextrins as components of formulations of the present invention are, 2-hydroxypropyl-beta-Cyclodextrin, 2-hydroxypropyl-gamma-Cyclodextrin, and hydroxyethyl-beta-Cyclodextrin. Cyclodextrin molecules (alpha, beta, or gamma) can have up to 3(n) substituents, where n is the number of glucopyranose units of the Cyclodextrin molecule. This is referred to as the degree of substitution (DS). The DS refers to substituents other than hydrogen; substituents may be all of one kind or mixed. Non-integer degrees of substitution occur as weighted averages are used to describe substitutional variability. See, e.g., Volume 3 (cyclodextrins) of the 11 Volume Collection "Comprehensive Supramolecular Chemistry", available through Elsevier Science Inc., 660 White Plains road, Tarrytown, N.Y., 10591-5153 USA. *See, also,* Pitha, Josef, U.S. Patent No. 4,727,064, *Pharmaceutical Preparations Containing Cyclodextrin Derivatives;* Muller, B.W., U.S. Patent No. 4,764,604, *Derivatives of Gamma Cyclodextrins;* Yoshida, A., et al., (1988) Int. Pharm, Vol. 46, p. 217: Pharmaceutical Evaluation

Of Hydroxy Alkyl Ethers Of B-Cyclodextrins; Muller, B. W., (1986). J. Pharm Sci. 75, No 6, June 1986: Hydroxypropyl-B-Cyclodextrin Derivatives: Influence Of Average Degree Of Substitution On Complexing Ability And Surface Activity; Irie, T., et al., (1988) Pharm Res., No 11, p. 713: Amorphous Water- Soluble Cyclodextrin Derivatives: 2-hydroxyethyl, 3-hydroxypropyl, 2-hyroxyisobutyl, and carboxamidomethyl derivatives of B-cyclodextrin.

Hydroxypropyl-B-Cyclodextrin (hydroxypropyl cyclodextrin) (HPCD) is itself very soluble in water (greater than 500 mg/ml at room temperature). Dr. Joseph Pitha of the NIH has experimentally evaluated many of the uses of this cyclodextrin derivative and found that it can be conveniently applied to cell cultures and membrane preparations. It is also observed that HPCD is non-toxic after IP and IV administration to different rodent species. The maximum human dose of HPCD given parenterally was approximately 500 mg/kg iv given continuously as a 5 percent aqueous solution to one individual for four days; no adverse clinical effects were reported. Pitha, Josef, et al., (1988) Life Sciences. 43, No 6, 493-502: Drug Solubilizers To Aid Pharmacologists: Amorphous Cyclodextrin Derivatives.

Compounds described herein are effectively formulated in compositions which generally comprise an effective amount of at least one α, β unsaturated aryl sulfone, water, and at least one chemically modified cyclodextrin. Embodiments of formulations of the present invention are preferred which comprise about 20% to about 60% w/v chemically modified cyclodextrin(s). Formulations described herein are preferred which have a pH within a range of about 7.6 to about 9.2. High pH, e.g., about 8 to about 9, or higher, is preferred. Particularly preferred compositions for the present invention comprise about 20% to about 60% w/v chemically modified cyclodextrin(s). Most preferred compositions for the present invention comprise about 30% to about 50% w/v chemically modified cyclodextrin(s). An example compositions of the present invention comprises at least one chemically modified cyclodextrin selected from the group consisting of 2-hydroxypropyl-beta-Cyclodextrin, 2-hydroxypropyl-gamma Cyclodextrin, and hydroxyethyl-beta-Cyclodextrin, preferably in an amount of about 30% to about 50% w/v, an effective amount of at least one α, β unsaturated aryl sulfone, i.e., (E)-4-carboxystyryl-4-chlorobenzylsulfone sodium salt (ON.1210.Na), and water. 2-hydroxypropyl-beta-Cyclodextrin, (Hydroxypropyl-B-Cyclodextrin) (hydroxypropyl cyclodextrin) (HPCD) is a preferred chemically modified cyclodextrin for use in compositions of the present invention. Shelf-stable aqueous compositions for the present invention, for dilution prior to parenteral administration for the protection of normal cells from toxicity of ionizing radiation or to mitigate the effects of accidental/intentional exposure to life-threatening levels of irradiation, comprise, for example, an effective amount of at least one α, β unsaturated aryl sulfone, and about 35% to about 45% (e.g., about 40% w/v) w/v chemically modified cyclodextrin.

Preferred shelf-stable compositions of the present invention, for dilution prior to administration, comprise between about 10 mg/ml to about 90 mg/ml of at least one α, β unsaturated aryl sulfone and at least one chemically modified cyclodextrin. Particularly preferred compositions of the present invention comprise between about 20 mg/ml to about 80 mg/ml of at least one α, β unsaturated aryl sulfone. Example compositions of the present invention comprise between about 30 mg/ml to about 50 mg/ml (e.g., about 40mg/ml) of at least one α, β unsaturated aryl sulfone (e.g., (E)-4-carboxystyryl-4-chlorobenzylsulfone sodium salt (ON.1210.Na)); and, about 30% to about 50% w/v (e.g., about 40%) chemically modified cyclodextrin (e.g., hydroxypropyl cyclodextrin); wherein the composition has a pH within the range of about 7.6 to about 8.5 (e.g., about 7.9).

Preferred formulations described and claimed herein have significantly increased the solubility of the radioprotective α, β unsaturated aryl sulfone, ON.1210.Na, for to allow concentrated formulations of the present invention, wherein upon dilution, the drug is physically stable for at least about 24 hours.

## IV. FORMULATION STUDIES

**ON.1210.Na**, a sodium salt of a chlorobenzylsulphone, is an example of an efficacious radiation protectant drug. ON.1210.Na is particularly shown to protect during life-threatening levels of radiation exposure. Described herein are shelf stable parenteral formulations of this and related compounds for therapeutic administration to patients.

A stability indicating HPLC assay is used during the preformulation studies. Preformulation studies include determination of microscopic and macroscopic properties, partition coefficient, pKa, pH-solubility, pH-stability, solid-state characterization and solid-state stability. XRD and thermal analyses re used to characterize the solid and solid-state stability. The solid-state stability as well as the pH stability of the drug is carried out at 75˚C.

Microscopic and XRD data reveals that the drug is crystalline having an irregular plate like crystals. The drug has a low octanol:water partition coefficient (1.28-2.87). The equilibrium solubility of the drug at pH 4.0, 5.0, 6.0, 7.4, 8.0, 9.0 is 0.000154, 0.0379, 0.715, 11.09, 16.81, 23.3 mg/mL, respectively. The pKa calculated from a pH-solubility study is 2.85 ± 0.6. The pH-stability profile of the drug indicats better stability at neutral and biological pH but degradation is rapid under acidic condition. The degradation followed a first-order rate. The accelerated solid-state stability of the bulk drug substance shows no evidence of degradation.

The solubility of the drug can be significantly increased with increase in pH. Also, the stability of the drug can be enhanced by buffering the aqueous solutions around pH 7. The drug is quite stable in an aqueous environment rendering itself for the development of a shelf stable formulation.

**Table 5: Saturated Solubility of ON.1210.Na in Different Solvent Systems**

| Solvents used | Solubility (mg/ml) | pH of solution | Dilution | Remarks |
|---|---|---|---|---|
| 20 % cyclodextran (Feb 4th, 24 hours of equilibration) | 62 | 8.26 | | |
| 10 % cyclodextran | 46 | 8.14 | | |
| 5% cyclodextran | 39 | 8.32 | | |
| (Feb 5th, 48 hours of equilibration) | | | | |
| 20 % cyclodextran | 58 | 8.35 | | |
| 10 % cyclodextran | 41 | 8.41 | | |
| 5% cyclodextran | 32 | 8.52 | | |
| Water | 11.8 | 8.40 | | |
| 10% PEG400:water | 36 | 8.24 | 1:4 diluted with PBS (the filtered sol was diluted with PBS) | Solution turns hazy after 24 hrs |
| 25 % PEG400:water | 39 | 8.01 | | |
| 50 % PEG400:water | 40 | 7.53 | | |
| 50% PEG400:Buffer (0.07M) | 23.4 | 8.2 | | |
| 50% PEG400:Buffer (0.01M) | 20.8 | 8.2 | | |
| 100 % PEG 400 (12g/kg) | 26 | 8.34 | | |
| 100 % ethanol | 0.9 | 7.94 | | |
| 50 : 50 PEG400 : ethanol | 37 | 8.46 | | |
| 20 % HPCD:20% PEG 400:60% water | 53.6 | 8.2 | | |
| 20 % HPCD:20% PEG 400:60% water | 53.6 | 8.2 | | |
| PEG400:Benzyl alcohol (50:50) | 51.6 | 8.19 | | |
| Propylene Glycol | 44.0 | 7.27 | | |
| PEG300 (IP 10g/kg) | 52.04 | 8.21 | | |
| 40% HPCD | 108.7 | 7.86 | | |
| For tween 80 in water | Solubility (mg/ml) | pH of solution | | |
| 0.25% | 10.5 | 8.87 | | |
| 0.5% | 10.3 | 8.74 | | |
| 1% | 10.1 | 8.82 | | |
| 2% | 9 | 8.78 | | |
| For Tween 80 in phosphate buffer | Solubility (mg/ml) | pH of solution | | |

(continued)

| Solvents used | Solubility (mg/ml) | pH of solution | Dilution | Remarks |
|---|---|---|---|---|
| 0.25% | 9.5 | 8.45 | | |
| 0.5% | 9.8 | 8.41 | | |
| 1% | 8.9 | 8.45 | | |
| 2% | 9.7 | 8.39 | | |
| Propylene glycol: water (50:50) | 41.65 | 8.53 | | |
| PEG 300: water (50: 50) | 30.6 | 4.5 | | |
| 1% (w/v) Povidone K 90 in water | 209.28 | 8.69 | | |
| 5% (w/v) Povidone K 90 in Water | 151.52 | 8.35 | | |
| NN-Dimethyl Acetamide:Water: PEG 400 (1:2:2) | 66.19 | 7.90 | | |
| 1 % PVP C 30 | 25.1 | 8.02 | | Saturated solution on filtration kept at RT turns hazy after 24 hrs |
| 2% PVP C 15 | 26.9 | 8.69 | | |
| 1% PVP C 15 | 26.9 | 8.86 | | Saturated solution on filtration kept at RT turns hazy after 24 hrs |
| PEG300:ETH (70:30) | 48.3 | 8.99 | | Saturated solution on filtration kept at RT turns hazy immediately |
| PEG400:Ethanol 70: 30 | 55.5 | 8.03 | | |
| DMA:WATER: Ethanol (1:1:1) | 46.4 | 8.11 | | |

**Table 6: Effect of Dilution on Solubility in different ON.1210.Na Formulations**

| Formulation | Concentration (mg/ml) | pH of solution | Dilution | Remarks |
|---|---|---|---|---|
| 50 mg/ml solution of ON.1210 in 40% HPCD | 44.99 | | 1. 1:4 (diluted with WFI)<br>2. 1:10 (diluted with WFI)<br>3. 1:4 (diluted with PBS) | No ppt. observed within 2 weeks<br>No ppt. observed within 2 weeks<br>No ppt. observed within 2 weeks |

(continued)

| Formulation | Concentration (mg/ml) | pH of solution | Dilution | Remarks |
|---|---|---|---|---|
| 50 mg/ml solution of ON.1210 in DMA: Water:PEG400 (1:2: 2) | 45.71 | | 1:4 (diluted with WFI)<br>1:10 (diluted with WFI)<br>1:4 (diluted with PBS) | Solution turns hazy after 24 hrs.<br>Solution turns hazy after 24 hrs.<br>No immediate turbidity, Solution turns hazy after 24 hrs. |
| 30 mg/ml solution of ON.1210.Na in PEG300:Ethanol (70:30) | 27.89 | | 1:4 (diluted with WFI)<br>1:10 (diluted with WFI)<br>1:4 (diluted with PBS) | Solution turns hazy immediately<br>Solution turns hazy immediately<br>No immediate turbidity but turbidity obs withir 24 hrs. |
| 30 mg/ml solution of ON.1210.Na in PEG400:Ethanol (70:30) | 27.48 | | 1:4 diluted with WFI<br>1:10 diluted with WFI<br>1:4 diluted with PBS | No immediate turbidity<br>No immediate turbidity<br>No immediate turbidity, also no turbidity after 24 hrs. |
| 50mg/ml ON. 1210.Na solution was prepared in PEG 400: Water (70:30) | 39.55 | | 1:4 (diluted with WFI)<br>1:10 (diluted with WFI)<br>1:4 (diluted with PBS) | Solution turns hazy immediately<br>Solution turns hazy immediately<br>No immediate turbidity, Solution turns hazy after 24 hrs. |
| 30mg/ml ON. 1210.Na solution in DMA:WATER: Ethanol (1:1:1) | | | 1:4 (diluted with WFI)<br>1:10 (diluted with WFI)<br>1:4 (diluted with PBS) | Solution turns hazy immediately<br>Solution turns hazy immediately<br>No immediate turbidity |

A study was conducted to develop and validate a stability indicating sensitive HPLC assay for ON.1210.Na for preformulation and formulation development. The isocratic system uses a mobile phase consisting of Acetonitrile:0.1% Trifluoroacetic acid in water (60:40 v:v) at a flow rate of 1 mL/min. A C-18 Gemini column (250 x 4.6 mm) is used and effluents are monitored at a 254 nm. Forced degradation is carried out by exposing ON.1210.Na to 0.1N HCl, 0.1N NaOH and 3% (v/v) hydrogen peroxide. The validation parameters include linearity, specificity, sensitivity, precision and accuracy.

Standard curves are linear in the concentration range of 0-500 μg/mL. The retention times of the drug and several degradation products were well within seven minutes. The Relative Standard Deviation (RSD) values for the within-day and day-to-day precision range from 0.4 to 2.5 % and 2.2 to 4.4 %, respectively. The RSD for accuracy measurement ranges from 0.85 to 1.7%. The critical level, the detection level and the determination level for this assay are 2.86 ± 0.67 μg/ml, 5.69 ± 0.67 μg/mL and 15.6 ± 1.79 μg/mL, respectively.

A sensitive and stability indicating HPLC method is developed and validated for ON.1210.Na. The force degradation, preformulation and formulation studies demonstrate the suitability of this method.

The first step in this process is the development of a stability indicating HPLC assay. The next phase is the validation of the assay. This HPLC assay is able to support preformulation, formulation development, and the stability studies of the bulk drug and formulated ON.1210.Na, for example.

Described herein is the development of the stability indicating assay, and the validation. The suitability of the assay is demonstrated by performing forced degradation studies on the bulk drug substance in solid state, and under acidic, alkaline, oxidizing and autoclaving conditions.

**HPLC ASSAY METHOD DEVELOPMENT AND VALIDATION**

A sensitive, highly specific and accurate stability indicating HPLC assay is developed and validated for the analysis of ON.1210.Na in aqueous samples. The following is a list of materials and equipment used to perform the method development and validation.

***Materials***

Trifluoroacetic Acid (Sigma, St. Louis, MO, USA); Acetonitrile, water (HPLC grade), (Fisher Scientific, NJ, USA) were used as received.

**Table 7: Equipment Used**

| | |
|---|---|
| HPLC System | HPLC system consisted of a pump (Model LC-600) programmed by a system controller (Model SCL-6B) |
| Detector | UV-Visible spectropliotometric detector (model SPD-6AV) |
| PDA | Shimadzu, SPD-M10AVP diode Array detector |
| Column | Gemini 5 μ-C-18 110A column (250x4.6 mm, SN# 26411-15, Phenomenex, CA, USA). |
| Autoclave | Tuttauer, Model: 2390E |
| Oven | Stabeltherh, Model: 4L543H |
| UV Spectrophotometer | UV 1700 PHARMASPEC model spectrophotometer |

**Ultraviolet Scan of ON.1210.Na**

An Ultraviolet (UV) scan of ON.1210.Na is obtained with a 10μg/mL of solution of ON.1210.Na in water using a UV 1700 PHARMASPEC model spectrophotometer (Shimadzu, Japan). The UV scan is shown in Figure 8. The UV spectrum shows two distinct maxima. The $\lambda_{max}$ for ON.1210.Na in this solvent is found to be at 281 nm and the second maxima occurs at 216 nm.

**HPLC METHOD DEVELOPMENT**

The initial HPLC method development process utilizes a gradient system with the following mobile phase composition:

Mobile phase A: Water containing 0.1% TFA
Mobile phase B: 100% HPLC grade Acetonitrile

The gradient used is a linear gradient from 0% B to 100% B over 30 minutes. A photodiode array detector (model SPD-M10AVP) is used at 3 wavelengths 230, 254 and 320 nm for monitoring the eluent. These three wavelengths are selected because of past experience with a similar analog ON.1910. The column used is a C-18 Gemini column (Phenomenex, CA). The flow rate is 1.0 mL/min. The resulting retention time of ON.1210.Na is approximately 23 minutes. Based on the data, this system is considered suitable for the detection of the parent compound with the other impurities present in the compound. Representative chromatograms of ON.1210.Na obtained at 254 nm is shown in Figure 9 (a-d). The chromatograms are presented in descending order.

The gradient system indicates that the retention time of the drug is more than 20 minutes and the degradation products during a forced degradation with 0.1N NaOH can be well separated and detected using the reversed phase system. Optimization of the mobile phase composition for the isocratic system is then undertaken.

## Development of an isocratic system

The next objective of the method development process is to reduce the run time without sacrificing the resolution between ON.1210.Na and its impurities/degradation products. Hence, the efforts are focused on developing an isocratic system utilizing the various combinations of acidified water and acetonitrile as the mobile phase. Three mobile phase composition are selected. They are: 20:80; 40:60 and 60:40 (v/v) of acetonitrile:water containing 0.1% TFA. The retention time ofON.1210.Na in the first two mobile phase compositions are more than 8 minutes, whereas with 60:40 (ACN: water) composition, the retention time is approximately 5 minutes at a flow rate of 1 mL/min. The resolution between ON.1210.Na and its three minor impurities is also good. A representative chromatogram is shown in Figure 10. The current lot (ON062604-1210Na) of ON.1210.Na shows a HPLC purity of more than 99%, with three other major impurities eluting at the retention times of 4.0, 6.0 and 6.6 minutes. Therefore, the third mobile phase composition is selected for further evaluation.

## Selection of the wavelength for analysis

A known concentration of the ON.1210.Na solution (100 μg/mL) is analyzed at three different wavelengths (230, 254 and 320 nm) and the absolute peak area at these wavelengths are determined and is reported below in Table 8.

**Table 8: Peak Area Response as a Function of Wavelength**

| Wavelength | Conc. (μg/mL) | PEAK AREA |
|---|---|---|
| 230 | 100 | 543548 |
| | | |
| 254 | 100 | 772519 |
| | | |
| 320 | 100 | 17618 |

The above results clearly indicate that analysis of the column effluents at 254nm is more sensitive as compared to the other two wavelengths. However, the sensitivity can be further improved if the analysis is performed at 281 nm. Before finalizing the optimal wavelength, two standard curves of ON.1210.Na are generated by analyzing the standard solutions at 230 and 254 nm. Linearity is superior at 254 rather than 230nm. Therefore, 254 nm is selected as the wavelength for monitoring all the future samples of ON.1210.Na.

## Forced Degradation studies

In order to establish the specificity of this assay procedure several forced degradation studies were conducted as follows:

### *Autoclaving of aqueous solution*

The ON.1210.Na sample is prepared in HPLC grade water at a concentration of 200 μg/mL. All the peaks detected in the chromatogram at time zero are noted with their corresponding retention time and absolute peak areas. The solution is crimped into a 5mL glass vial and autoclaved. After autoclaving, the sample is filtered through a 0.1 μ filter and 20 μL of the filtered sample is analyzed. The absolute peak area of the peak after autoclaving and appearance of any additional peaks due to possible degradation is shown in Table 9.

**Table 9: Effect of Autoclave on Aqueous Solution of ON.1210.Na**

| Sample Description | Peak detected at the retention time (min) | Peak area at time zero (before autoclaving) | Peak Area after autoclaving | Appearance Of any new peaks |
|---|---|---|---|---|
| ON.1210.Na in aqueous solution | 4.08 | 1938 | 1995 | NO |
| | 4.97 | 1299297 | 1303758 | |
| | 6.07 | 1346 | 1382 | |
| | 6.63 | 776 | 983 | |

The data in Table 9 demonstrates that there is no change in the peak area of ON.1210.Na before and after autoclaving. Also, there are no additional peaks detected. Hence, it seems that the aqueous solution is stable under autoclaving conditions.

***Forced degradation with 0.05N HCl***

One ml of the 500 μg/mL standard solution is mixed with 1 mL of 0.1 N HCl. The sample is cloudy in appearance and filtered prior to HPLC analysis. All the peaks detected in the chromatogram at time zero are noted with their corresponding retention time and absolute peak areas. The solution is crimped into a 5 mL glass vial and autoclaved. After autoclaving, the sample is filtered through a 0.1 μ filter and 20 μL of the filtered sample is analyzed by HPLC. The absolute peak areas of the peak after autoclaving, and appearance of any other peaks due to possible degradation are noted. The results are depicted in Table 10.

There is less than 10% degradation upon autoclaving ON.1210.Na in presence of strong acid. However, it does degrade and the current HPLC method is able to detect three additional peaks.

**Table 10: Forced Degradation of ON.1210.Na with 0.05N HCl**

| Sample Description | Peak detected at the retention time (min) | Peak area at time zero (before autoclaving) | Peak Area after autoclaving | Appearance Of any new peaks |
|---|---|---|---|---|
| ON.1210.Na in 0.05N HCl | 4.14 | 1884 | 2275 | |
| | 4.53 | 2022 | 1536 | |
| | 5.05 | 160391 | 148189 | |
| | 6.3 | 1770 | not integrated | |
| | *4.56 | - | 581 | Extra peak |
| | *5.458 | - | 1090 | Extra peak |
| | *6.52 | - | 440 | Extra peak |
| *appearance of extra degradation peaks after autoclaving | | | | |

***Forced degradation with 0.05N NaOH***

One ml of the 500 μg/mL standard solution is mixed with 1 mL of 0.1 N NaOH. The sample is clear in appearance. All the peaks detected in the chromatogram at time zero are noted with their corresponding retention time and absolute peak area. The solution is crimped into a 5mL, glass vial and autoclaved. After autoclaving, the sample is filtered through a 0.1 μ filter and 20 μL of the filtered sample is analyzed. The absolute peak area of the peaks after autoclaving and appearance of any other peaks due to possible degradation are noted. The results are depicted in Table 11.

**Table 11: Forced Degradation of ON.1210.Na with 0.05N NaOH**

| Sample Description | Peak detected at the retention time (min) | Peak area at time zero (before autoclaving) | Peak Area after autoclaving | Appearance Of any new peaks | % change in peak area |
|---|---|---|---|---|---|
| ON.1210.Na in 0.05 N NaOH | 4.12 | 4328 | 72158 | | 1567 |
| | 5.03 | 2008425 | 1558358 | | -22.4 |
| | 6.13 | 2149 | 1523 | | -21.9 |
| | 6.71 | 1233 | Not integrated | | |
| | *3.38 | - | 397067 | Extra peak | |
| | *6.07 | - | 1523 | Extra peak | |
| | *6.48 | - | 265 | Extra peak | |
| *appearance of extra degradation peaks after autoclaving. | | | | | |

Significantly more degradation is present under alkaline condition. Formation of a major degradation product is present which elutes at the retention time of 3.3 minutes.

***Stability of the ON.1210.Na (solid)***

A solid drug sample (ON1210), after autoclaving in a crimped vial, is also evaluated for any degradation using the developed HPLC method. The sample, after autoclaving, is used to make a standard solution of 400 μg/mL and injected onto HPLC. The results indicate no reduction in the Area Under the Curve (AUC) of the parent peak, and also no additional peaks are detected. Based on this observation, it seems that the bulk drug ON.1210.Na is stable under the autoclaving conditions.

***Forced degradation with 1.5% (v/v) hydrogen peroxide solution***

In this study, 30% of the hydrogen peroxide is diluted to 3% (v/v) with water. One ml of the 500 μg/mL standard solution is mixed with 1 mL of 3% hydrogen peroxide; then incubated at 50°C over a period of two hours. All the peaks detected in the chromatogram at different time points are noted with their corresponding retention time and absolute peak areas. The chromatograms are run for 30 minute periods. The results are depicted in Table 12.

**Table 12: Forced Degradation of ON.1210.Na with Hydrogen Peroxide**

| Sample Description | Peak detected at the retention time (min) | Peak area after one hour of incubation | Peak Area after two hours of incubation | % change in peak area |
|---|---|---|---|---|
| ON.1210.Na in 1.5% hydrogen peroxide | 1.92 | 315 | 1859 | 490 |
| | 2.56 | 2750467 | 2700331 | -1.80 |
| | 3.14 | 5564 | 29089 | 423 |
| | 3.36 | 3361 | 6411 | 90.7 |
| | 3.63 | 9171 | 7851 | -14.4 |
| | 4.22 | 2002560 | 1845952 | -7.82 |
| | 4.95 | 2917 | 5824 | 99.7 |
| | 5.39 | 445 | 706 | 58.7 |

All of the above forced degradation studies indicated that the HPLC method developed for ON.1210.Na is stability indicating. The peak of ON.1210.Na is from a single component and none of the degradation products are co-eluting.

## HPLC METHOD VALIDATION

### *Standard Solutions and sample preparation*

The stock standard solution (1000 μg/ml) is prepared by dissolving 0.10 g of ON.1210.Na in 100ml 60:40 (v/v) of ACN:water without TFA. The apparent pH of the solution before addition of the drug is 7.52. Various standard solutions (20-500 μg $ml^{-1}$) are then prepared by diluting the above stock solution with mobile phase without TFA to yield nominal concentrations over a range 10-500 μg/ml as shown in the table below.

**Table 13: Preparation of Standard Solutions**

| Volume of the stock solution used (ml) | Diluted to the final volume (ml) with mobile phase without TFA | Final concentration (μg /ml) |
|---|---|---|
| 0 | 50 | 0 |
| 0.5 | 50 | 10 |
| 5 | 100 | 50 |
| 5 | 50 | 100 |
| 5 | 25 | 200 |
| 5 | 10 | 500 |

Three quality control samples are also prepared from the same stock solution (1000 μg /ml) for the accuracy measurement as shown in table below.

**Table 14: Preparation of Quality Control Samples**

| Volume of the stock solution used (ml) | Diluted to the final volume (ml) with mobile phase without TFA | Final concentration (μg /ml) |
|---|---|---|
| 2 | 100 | 20 |
| 2.5 | 10 | 250 |
| 4 | 10 | 400 |

The standard solution or the sample to be analyzed (200 μl) is placed in sample vial in an auto injector and an aliquot (20 μl) is analyzed by HPLC.

The mobile phase is prepared by mixing 600 ml of acetonitrile and 400 ml of HPLC water containing 0.1% (v/v) TFA. The solution is filtered through a 0.45- μm MAGNA Nylon, 47-mm filter (MSI, MA, USA). The filtered mobile phase is then sonicated for one hour for degassing.

### *Assay validation*

*Linearity*

Standard curves are constructed by plotting peak area versus concentration of the drug. Standard curves for ON.1210.Na are linear over the concentration range of 10-500 μg/ml. The equation of the standard curve relating the peak area (PA) to the drug concentration (C in μg /ml) in this range is PA = 7372.3C + 0.14556, $R^2$>0.999.

*Precision*

Within-day precision of the assay is determined by analysis of replicate (n=4) samples of six different concentration on the same day. To determine day-to-day precision, the same solutions on five different days are analyzed during a period of 12 days. The variability in the peak-area at each concentration are presented in Tables 15 and 16. Within-day and day-to-day RSD values for the ON.1210.Na assay range from 0.4 to 2.5 %. During this period, the stock solution and standard solutions are stored under room temperature (23°C). The day-to-day precision RSD values for ON.1210.Na are 2.2 to 4.4 %.

*Accuracy*

Three quality control samples (QCs) for ON.1210.Na are placed at room temperature at 23˚C over a period of 15 days. These samples are analyzed five times during this time and the accuracy of the assay is determined by comparing the measured concentration to its nominal value. The results of the study are depicted in Table 16. The RSD's for ON.1210.Na ranged from 0.85 to 1.7 %.

*Sensitivity*

The lowest limit of reliable assay measurement criteria described by Oppenheimer et al[2] is used to determine the sensitivity parameters. Six different standard curves are used in this calculation. The critical level is defined as the assay response above which an observed response is reliably recognized as detectable. This value is also considered as the threshold value, defining detection. If the measured value exceeds this value then the presence of analyte is detected, otherwise it is not. The critical level is 2.86 ± 0.33 $\mu$g $ml^{-1}$ (Mean ± S.D.). The detection level is the actual net response, which may a priori be expected to lead to detection. This is the lowest better value of the true concentration that is "nearly sure" to produce a measured value that results in detection. The detection level is 5.69 ± 0.67 $\mu$g $ml^{-1}$ (Mean ± S.D.). The determination level is the concentration at which the measurement precision will be satisfactory for quantitative determination. The determination level is 15.6 ± 1.79 $\mu$g $ml^{-1}$ (Mean ± S.D.) for a level of precision of 10% RSD.

**Stability of the standard solutions**

The standard solutions are very stable over a period of 12 days when kept at room temperature. The RSD (less than 2.5%) of the slope of the standard curves for the day-to-day precision measurement over 12 day's storage data supports the claim. No additional peaks are detected in the standards when kept over 12 days at room temperature.

**Table 15: Within-day and day-to-day analytical precision for ON.1210.Na**

| Within-day | | | Day-to-day | |
|---|---|---|---|---|
| Concentration (µg/ml) | *Mean peak-area | RSD (%) | **Mean peak-area | RSD (%) |
| 0 | 0 | 0 | 0 | 0 |
| 10 | 79531.0 | 0.8 | 82255.8 | 4.4 |
| 50 | 385264.5 | 2.5 | 393338.8 | 2.5 |
| 100 | 759900.8 | 0.6 | 772519.5 | 2.6 |
| 200 | 1498462.0 | 0.4 | 1522404 | 2.1 |
| 500 | 3666046.0 | 0.5 | 3723656 | 2.1 |
| Slope | 7321.1 ± 37.1 | 0.5 | 7434.1 ± 156.1 | 2.1 |
| *n=4<br>**n=6, over a period of 12 days | | | | |

**Table 16: Accuracy in the analysis of ON.1210.Na in quality control samples**

| Actual Concentration (µg /ml) | Measured Concentration* (µg /ml) | Accuracy** |
|---|---|---|
| 20 | 19.5±0.33 | 97.7±1.65 |
| 250 | 254.1±2.22 | 101.6±0.89 |
| 400 | 398.3±3.37 | 99.6±0.84 |
| *Mean ± S.D.; n=5<br>**Accuracy = (Measured conc./actual conc.)x100 | | |

**Octanol-water Partition Coefficient**

The octanol-water partition coefficient of the drug is determined at 25°C. Equal volumes (10 ml) saturated solution of ON.1210.Na in n-octanol and HPLC water are equilibrated at 25°C in a shaking water bath for 48 hours. The pH of the aqueous phase is also determined. At a predetermined time, a known volume of both the organic and aqueous phase is collected via a filtered needle. Subsequently, the concentration of ON.1210.Na is determined by HPLC after adequate dilution.

***pH Solubility Testing***

The solubility of ON.1210.Na salt is tested in McIlvaine type buffer with 0.1M ionic strength. The buffers are prepared using the recipe mentioned in Table 17 The final pH is adjusted using 0.1N sodium hydroxide or 0.1N hydrochloric acid until the pH is within ±0.1 pH units of the target pH. The results of the study are presented in Table 17. The pKa of the drug calculated from pH-solubility study is depicted in Table 19.

***pH Stability Testing***

The drug is dissolved in different pH buffers as outlined in Table 20. One hundred mL of each solution is prepared in a volumetric flask. Each solution is filtered through a 0.22um filter. Approximately, four mL of the above solution is placed in 5 ml ampoules and sealed by a propane torch. The pH of the solution is determined at time zero and at each time interval. All the ampoules are placed in a 75°C constant temperature oven. Any color changes or precipitation in the solution during the sample collection is visually observed. Concentration of the drug is determined at time zero and other time intervals by HPLC. The sampling schedules for the stability data are shown in Table 21. The concentration of the drug at different time points is shown in Table 22.

**Thermal analyses**

A differential scanning calorimeter (DSC) (model DSC-50, Shimadzu, Kyoto, Japan) and a thermogravimetric analyzer (TGA) (model TGA-50, Shimadzu, Kyoto, Japan) are connected to a thermal analysis operating system (TA-50WS, Shimadzu, Kyoto, Japan). The heat of fusion is calibrated using indium (purity 99.99%; mp 156.4; ΔH 6.8 mcal/mg). The sample to be analyzed (5-10 mg) by DSC is crimped non-hermetically in an aluminum pan and heated from 30 to 400°C at a rate of 10°C/min under a stream of nitrogen (flow rate of 20 mL/min). For the thermogravimetric analysis (TGA), approximately 10 mg of the sample is weighed into aluminum pans and heated from 30 to 400°C at a heating rate of 10°C/min under nitrogen purge.

**Powder X Ray Diffraction Studies**

The ON. 1210 free acid, sodium salt and the precipitate from filtered saturated solution of ON.1210.Na kept at refrigerated temperature are analyzed for its crystallinity by XRPD. The instrument used is a Siemens D5005. The samples are analyzed at room temperature with a scan range (2θ) of 5° to 40° (Step scan, step size of 0.05°, dwell time of 1 second). The sample holder is a Zero background holder.

**RESULTS**

**Octanol-water partition coefficient**

The octanol-water partition coefficient of the drug is determined at 25°C using equal volumes (10 ml) of saturated solution of ON.1210.Na in n-octanol and HPLC water. Similarly, the partition coefficient is determined using simulated gastric and intestinal fluid without enzymes. The octanol-water partition coefficient is determined using the following equation:

$$\textbf{Octanol-water partition coefficient} = \frac{\textit{Concentration of drug in Octanol Phase}}{\textit{Concentration of drug in aqueous phase}}$$

The octanol-water partition coefficient determined in triplicate samples is determined to be 1.28 to 2.87. The pH of the aqueous phase is 8.1.

The partition coefficient using simulated gastric fluid can not be determined because of the extremely low solubility of ON.1210.Na at a pH of 8.1. The pH of the simulated gastric fluid is 1.62. The partition coefficient using simulated intestinal fluid is 0.74 to 2.1. The data obtained from partition coefficient determination is found to be highly variable. Hence, further investigation is necessary to identify this variability. The pH of the simulated intestinal fluid is 7.83.

***pH Solubility Testing***

The solubility of the ON.1210.Na salt is tested in a McIlvaine type buffer with 0.1M ionic strength. The buffers are prepared using the recipe mentioned in Table 16 using various volumes of 0.15 M citric acid monohydrate, and 0.2 M sodium phosphate dibasic, 12 hydrate. The final pH is adjusted using 0.1N sodium hydroxide or 0.1N hydrochloric acid until the pH is within ±0.1 pH units of the target pH.

**Table 17: Preparation of McDvaine Buffer with Constant Ionic Strength**

| Target pH | Phosphate | Citric Acid | Final Volume* | Final pH |
|---|---|---|---|---|
| 4.0 | 38.5 | 40.7 | 150 | 4.06 |
| 5.0 | 51.5 | 32.0 | 250 | 5.07 |
| 6.0 | 63.0 | 24.7 | 340 | 6.04 |
| 7.4 | 90.5 | 6.1 | 480 | 7.46 |
| 8.0 | 97.0 | 1.9 | 550 | 8.06 |
| 9.0 | 97.0 | 1.9 | 550** | 9.03 |
| *Initially 100 mL of solution was prepared. The solutions with pH 4.0-8.0 all have a calculated ionic strength of greater than 0.1M, thus the solutions were diluted with deionized water to obtain the final ionic strength of 0.1M. **The final pH was adjusted to 9 by addition of 0.1N NaOH solution. | | | | |

The samples for the pH solubility study are prepared by adding 10-20 mg of ON.1210.Na salt to a 4 mL vial. Then 1 mL of the appropriate pH buffer is added to the vial. The solution is mixed on a vortex genie for approximately 30 seconds. If the solution looks clear, additional drug is added. No additional drug is added to solutions which appear to be clear at a concentration of greater than 50 mg/mL. The study is done in duplicate.

Once the solutions are prepared, the initial pH of the samples are measured. The solutions are then protected from light, and placed on a shaker. The solutions are agitated for 24 to 96 hours at ambient conditions. The vials are then removed from the orbital shaker, and the final pH of the solutions is measured for each sample. The solutions are then filtered, and analyzed by HPLC.

The pH solubility data is shown in Table 18 and the pH solubility profile is shown in Figure 11.

**Table 18: pH solubility data for ON.1210.Na**

| pH | Solubility (mg/mL) |
|---|---|
| 4 | 0.000154 |
| 5 | 0.0379 |
| 6 | 0.715 |
| 7.4 | 11.09 |
| 8 | 16.81 |
| 9 | 23.3 |

**Determination of pKa from saturated solubility studies at different pH**

The pKa of the weakly acidic drug is determined from pH-solubility data using the equation provided below.

$$\log\left(\frac{c_s}{c_0}-1\right)=pH-pK_a$$

Where $C_s$ = observed solubility and $C_0$= intrinsic solubility of the compound,
The calculations are shown in Table 19.

**Table 19: Calculation of pKa from pH-solubility data**

| pH | Observed Solubility ($C_s$) (mg/mL) | Observed Solubility ($C_s$) ug/ml | ($C_s$/$C_0$) | ($C_s$ /$C_0$)-1 | log($C_s$/$C_0$)-1 | pKa | Mean pKa | SD |
|---|---|---|---|---|---|---|---|---|
| 4 | 0.000154 | 0.154 | 1 | 0 | | | 2.85 | 0.5856 |
| 5 | 0.0379 | 37.9 | 246.1039 | 245.103 | 2.38935 | 2.61 | | |
| 6 | 0.715 | 715 | 4642.857 | 4641.85 | 3.666692 | 2.33 | | |
| 7.4 | 11.09 | 11090 | 72012.99 | 72011.9 | 4.857405 | 2.54 | | |
| 8 | 16.81 | 16810 | 109155.8 | 109154. | 5.038043 | 2.96 | | |
| 9 | 23.30 | 23300 | 151298.7 | 151297. | 5.179832 | 3.82 | | |

Since the drug is weakly acidic, its intrinsic solubility is assumed to be the solubility at a very low pH (pH=4) where the contribution due to ionization is minimal. The intrinsic solubility is taken as 0.000154mg/mL in this case. As a result, the pKa determined by the saturated solubility study was 2.85 ± 0.59.

**pH Stability Testing**

The drug is dissolved in different pH buffers as outlined in Table 20. One hundred mL of each solution is prepared in volumetric flask. Each solution is filtered through 0.22um filter. Approximately, four mL of the above solution is placed in 5 ml ampoules and sealed by a propane torch. The pH of the solution is determined at time zero and at each time interval. All the ampoules are placed in a 75°C constant temperature oven. Any color changes or precipitation in the solution during the sample collection is visually observed. Concentration of the drug is determined at time zero and other time intervals by HPLC. The sampling schedules for the stability data are shown in Table 21. The pH and drug content of each sample are determined. The concentration at each time point and their pH is provided in Table 22.

**Table 20. Concentration of the drug used for the pH stability studies at 75°C**

| pH | Concentration of the solution used | Amount weighed into 100 ml of solution pH |
|---|---|---|
| 4 | 4 μg/ml | 0.4 mg |
| 5 | 100 μg/ml | 10 mg |
| 6 | 200 μg/ml | 20 mg |
| 7.4 | 500 μg/ml | 50 mg |
| 8 | 500 μg/ml | 50 mg |
| 9 | 500 μg/ml | 50 mg |

**Table 21: Sampling Schedule for pH Stability Samples**

| Sample | Days | | | | | | |
|---|---|---|---|---|---|---|---|
| | 0 | 1 | 3 | 5 | 10 | 17 | 28 |
| | 35 | 42 | 54 | | | | |
| pH 4.0 | X | X | X | X | X | X | X |
| | X | X | X | | | | |

(continued)

| Sample | Days | | | | | | |
|---|---|---|---|---|---|---|---|
| | 0 | 1 | 3 | 5 | 10 | 17 | 28 |
| | 35 | 42 | 54 | | | | |
| pH5.0 | X | X | X | X | X | X | X |
| | X | X | X | | | | |
| pH6.0 | X | X | X | X | X | X | X |
| | X | X | X | | | | |
| pH7.4 | X | X | X | X | X | X | X |
| | X | X | X | | | | |
| pH8.0 | X | X | X | X | X | X | X |
| | X | X | X | | | | |
| pH9.0 | X | X | X | X | X | X | X |
| | X | X | X | | | | |

**Table 22. Concentration of the ON.1210.Na (μg/mL) at different time points as determined by HPLC during the stability study.**

| pH | 0 hours | 1day | 3 days | 5 days | 10 days | 17 days | 28 days | 35 days | 42 days | 54 days |
|---|---|---|---|---|---|---|---|---|---|---|
| 4 | 2.7047 (4.03) | 2.659039 (4.3) | 2.6331 (4.12) | 2.7344 (4.5) | -0.72547 (4.54) | -0.681844 (4.5) | -0.1894 (3.61) | No detection (3.85) | 0.11707 (3.84) | 0.161366 (3.74) |
| 5 | 4.4577 (5.03) | 4.394361 (5.18) | 4.4295 (5.11) | 4.4714 (5.14) | 1.01704 (5.2) | 0.838165 (5.21) | 1.3340 (4.71) | 2.8137 (4.93) | 1.30 49 | 0.953869 (4.76) |
| 6 | 22.39 (6.02) | 22.29934 (6.12) | 22.35858 (6.21) | 22.4945 (6.14) | 19.0863 (6.17) | 18.15392 (6.21) | 17.5401 (5.81) | 17.3979 (5.92) | 15.99869 (5.94) | 14.7174 (5.90) |
| 7.4 | 485.952 (7.41) | 485.8206 (7.42) | 481.4637 (7.52) | 484.009 (7.44) | 473.2525 (7.42) | 466.5611 (7.41) | 434.55 98 (7.2) | 428.5503 (7.34) | 416.3353 (7.36) | 403.267 (7.31) |
| 8.0 | 494.8540 (8.01) | 493.8002 (8.08) | 485.8134 (8.04) | 486.042 (7.98) | 462.5487 (7.98) | 441.454 (8.02) | 398.9563 (7.81) | 249.7589** (7.33) | 378.4188 (7.36) | 353.5676 (7.9) |
| 9.0 | 475.7458 (9.02) | 463.3368 (9.1) | 436.1314 (9.12) | 422.51 (8.72) | 379.1650 (8.74) | 351.6448 (8.75) | 304.3917 (8.53) | 199.584 *** (8.46) | 300.1106 (8.45) | 267.741 (8.47) |

*number in the parenthesis represents the pH of the solution; **New degradation product with retention time 4.52 min; *** Two new degradation products with retention time of 4.52 and 3.6 minutes.

The data from the pH stability study is analyzed by plotting Log (conc.) versus time. The results are shown in Figure 12.

The degradation of ON.1210.Na seems to follow a pseudo first order reaction. The rate constant for the degradation is determined using a linear regression at each individual pH. The apparent first order rate constant as a function of pH are listed in Table 23.

**Table 23: First order rate constant for the degradation of ON.1210.Na as a function of pH at 75˚C**

| pH | $K_{obs}$ ($Days^{-1}$) at 75˚C |
|---|---|
| 4.0 | 0.0621 |
| 5.0 | 0.0295 |
| 6.0 | 0.0069 |
| 7.4 | 0.0037 |
| 8.0 | 0.0064 |
| 9.0 | 0.0104 |

The above data clearly shows that the stability of ON.1210.Na in aqueous buffers is dramatically improved as the pH is increased towards the neutral pH. However, further increase in pH might adversely affect its stability. Also, based on the above data it is expected that the aqueous formulations buffered around pH 7.0 might be shelf stable at room temperature.

**Solid-state stability of ON.1210.Na**

The stability of ON.1210.Na in the solid state is determined by exposing the drug at 75˚C over a prolonged time. The sample is crimped in a 10 mL injection vial and kept at the above temperature. The sample is collected after 15 days and one month and reconstituted with diluting solution and its concentration is determined by HPLC. Appearance of any additional peak in the chromatogram is also noted. A control sample is represented by ON.1210.Na not exposed to 75˚C and reconstituted to a known concentration with diluting solution. The results of this study are depicted in Table 24.

**Table 24: Solid-state stability of ON.1210.Na at 75˚C**

| Day after exposure | Theoretical conc. (ug/mL) | Determined conc. (ug/mL) | % change | Sample not exposed to Heating condition | Theoretical conc. (ug/mL) | Determined concentration (ug/mL) | % change |
|---|---|---|---|---|---|---|---|
| 15 | 270 | 286.2 | +6 | (control) | 250 | 242.7 | -2.92 |
| 30 | 270 | 334.9 | +24 | (control) | 250 | 232.3 | -7.1 |
| 45 | 270 | 302.0 | +11.8 | (control) | 250 | 249.8 | -0.44 |
| 54 | 200 | 229.2 | +14.6 | (control) | 200 | 203.5 | +1.75 |

The solid state stability data shows that there is a slight increase in potency of the compound as a function of time at 75˚C. This is probably due to the loss of moisture present in the sample. This was confirmed by determining the moisture content of the bulk ON.1210.Na. The result shows that there is about 14.5% of moisture in the sample which equates to 3 moles of water per mole of ON.1210.Na. The chromatograms showed no evidence of degradation of the sample.

**Thermal analyses**

A differential scanning calorimeter (DSC) (model DSC-50, Shimadzu, Kyoto, Japan) and a thermogravimetric analyzer (TGA) (model TGA-50, Shimadzu, Kyoto, Japan) are connected to a thermal analysis operating system (TA-50WS, Shimadzu, Kyoto, Japan). The heat of fusion is calibrated using indium (purity 99.99%; mp 156.4; ΔH 6.8 mcal/mg). The sample to be analyzed (5-10 mg) by DSC is crimped nonhermetically in an aluminum pan and heated from 30 to 400˚C at a rate of 10˚C/min under a stream of nitrogen (flow rate of 20 mL/min). For the thermogravimetric analysis

(TGA), approximately 10 mg of the sample is weighed into aluminum pans and heated from 30 to 400˚C at a heating rate of 10˚C/min under nitrogen purge. The DSC thermogram indicates two thermal events: (i) an endothermic peak at 50˚C possible due to desolvation or dehydration. This is further supported by the TGA thermogram which also indicates a weight loss exactly at the same temperature range, and (ii) the exothermic peak at 360˚C probably due to the melting of the drug with decomposition.

**Powder x-ray diffraction Studies**

The samples free acid (ON.1210), the salt (ON.1210.Na) and the precipitated sample from filtered saturated ON.1210.Na solution kept at refrigerated condition are analyzed using Siemens powder X Ray diffractometer under ambient conditions. The scan range (2θ) is from 5˚ to 40˚ (Step scan, step size of 0.05˚, with a dwell time of 1 second). The results are shown in Figures 14-15, along with the peak search report.

**Table 25: Peak Search Report for ON.1210 Acid**

| Peak Search Report (21 Peaks, Max P/N = 6.7)<br>PEAK: 11-pts/Parabolic Filter, Threshold=3.0, Cutoff=0.1%, BG=3/1.0, Peak-Top=Summit | | | | | | | |
|---|---|---|---|---|---|---|---|
| **2-Theta** | **d(Å)** | **BG** | **Height** | **I%** | **Area** | **I%** | **FWHM** |
| 6.048 | 14.6011 | 20 | 102 | 52.9 | 559 | 48.2 | 0.234 |
| 10.07 | 8.777 | 9 | 12 | 6 | 57 | 4.9 | 0.196 |
| 10.341 | 8.5478 | 9 | 12 | 6 | 38 | 3.3 | 0.131 |
| 13.424 | 6.5905 | 8 | 3 | 1.8 | 7 | 0.6 | 0.1 |
| 14.046 | 6.2999 | 10 | 14 | 7.3 | -40 | -3.4 | 0.05 |
| 15.002 | 5.9008 | 7 | 25 | 13.1 | 89 | 7.7 | 0.15 |
| 15.75 | 5.6219 | 6 | 23 | 11.8 | 100 | 8.6 | 0.187 |
| 17.144 | 5.1681 | 14 | 181 | 94.2 | 896 | 77.2 | 0.198 |
| 17.495 | 5.0651 | 16 | 192 | 100 | 1160 | 100 | 0.256 |
| 18.251 | 4.8569 | 18 | 97 | 50.2 | 401 | 34.6 | 0.177 |
| 18.827 | 4.7096 | 14 | 13 | 6.7 | 150 | 12.9 | 0.468 |
| 19.147 | 4.6316 | 16 | 13 | 6.9 | 35 | 3 | 0.105 |
| 20.197 | 4.393 | 10 | 24 | 12.4 | 124 | 10.7 | 0.221 |
| 20.519 | 4.325 | 11 | 16 | 8.4 | 124 | 10.7 | 0.326 |
| 23.65 | 3.7589 | 11 | 22 | 11.7 | 115 | 9.9 | 0.217 |
| 28.24 | 3.1576 | 11 | 21 | 10.7 | 137 | 11.8 | 0.283 |
| 29.291 | 3.0466 | 10 | 17 | 9 | 40 | 3.4 | 0.098 |
| 30.563 | 2.9226 | 8 | 26 | 13.7 | 188 | 16.2 | 0.304 |
| 32.797 | 2.7285 | 8 | 9 | 4.8 | 23 | 2 | 0.1 |
| 35.9 | 2.4994 | 6 | 10 | 5.2 | 37 | 3.2 | 0.149 |
| 39.383 | 2.286 | 6 | 11 | 5.5 | 28 | 2.4 | 0.107 |

**Table 26: Peak Search Report for ON.1210 Na (13 Peaks, Max P/N = 10.6)**

| PEAK: 11-pts/Parabolic Filter, Threshold=3.0, Cutoff-0.1%, BG=3/1.0, Peak-Top=Summit | | | | | | | |
|---|---|---|---|---|---|---|---|
| **2-Theta** | **d(Å)** | **BG** | **Height** | **I%** | **Area** | **I%** | **FWHM** |
| 6.606 | 13.3698 | 14 | 73 | 16 | 204 | 10.1 | 0.119 |
| 7.366 | 11.9924 | 12 | 16 | 3.6 | 26 | 1.3 | 0.063 |

(continued)

| PEAK: 11-pts/Parabolic Filter, Threshold=3.0, Cutoff-0.1%, BG=3/1.0, Peak-Top=Summit | | | | | | | |
|---|---|---|---|---|---|---|---|
| **2-Theta** | **d(Å)** | **BG** | **Height** | **I%** | **Area** | **I%** | **FWHM** |
| 8.815 | 10.024 | 8 | 156 | 34.1 | 535 | 26.4 | 0.146 |
| 9.544 | 9.2595 | 9 | 22 | 4.7 | 54 | 2.7 | 0.106 |
| 17.762 | 4.9896 | 11 | 25 | 5.6 | 25 | 1.3 | 0.05 |
| 19.756 | 4.4902 | 8 | 20 | 4.4 | 224 | 11.1 | 0.478 |
| 23.647 | 3.7595 | 10 | 16 | 3.6 | 33 | 1.6 | 0.086 |
| 24.153 | 3.6817 | 8 | 22 | 4.8 | 59 | 2.9 | 0.114 |
| 28.968 | 3.0799 | 9 | 456 | 100 | 2025 | 100 | 0.189 |
| 31.074 | 2.8757 | 8 | 31 | 6.9 | 28 | 1.4 | 0.05 |
| 31.453 | 2.842 | 6 | 107 | 23.5 | 332 | 16.4 | 0.132 |
| 33.765 | 2.6524 | 6 | 16 | 3.5 | 67 | 3.3 | 0.178 |
| 39.002 | 2.3075 | 11 | 282 | 61.9 | 1481 | 73.2 | 0.223 |

Figures 15 clearly shows that the sodium salt of ON.1210 is a crystalline material.

**Characterization of Precipitate**

The sodium salt has a tendency to form a supersaturated solution in water, Upon storage, the solution starts showing some precipitate. The precipitate is isolated, dried and characterized by HPLC, DSC, TGA and XRPD.

The precipitate is dissolved in acetonitrile water mixture and analyzed by HPLC. The precipitate elutes at the same time as the standard of ON.1210.Na suggesting that the precipitate is of ON.1210.

The precipitate is further characterized by DSC. A thermogram of the precipitate does not show the transition around 50˚C as is seen with the ON.1210.Na standard. However, it does show the same melting / decomposition around 360˚C.

The precipitate is further characterized by XRPD. The diffractogram shows most of the peaks that are observed with ON.1210.Na except the one observed at 8.815.

**Table 27: Peak search report of precipitated sample**

| Peak Search Report (6 Peaks, Max P/N = 10.9) PEAK: 11-pts/Parabolic Filter, Threshold=3.0, Cutoff=0.1%, BG=3/1.0, Peak-Top=Summit | | | | | | | |
|---|---|---|---|---|---|---|---|
| **2-Theta** | **d(Å)** | **BG** | **Height** | **I%** | **Area** | **I%** | **FWHM** |
| 9.52 | 9.2823 | 7 | 44 | 9.2 | 159 | 9.1 | 0.153 |
| 16.749 | 5.2891 | 6 | 23 | 4.7 | 116 | 6.7 | 0.216 |
| 21.83 | 4.068 | 6 | 24 | 4.9 | 101 | 5.8 | 0.182 |
| 26.461 | 3.3656 | 4 | 10 | 2.1 | 42 | 2.4 | 0.168 |
| 28.957 | 3.081 | 6 | 481 | 100 | 1742 | 100 | 0.154 |
| 38.981 | 2.3087 | 5 | 168 | 35 | 812 | 46.6 | 0.205 |

In conclusion, this drug, e.g., ON.1210.Na, is surprisingly demonstrated to be quite stable in an aqueous environment at biological pH. Therefore, it can be formulated as an efficacious shelf stable parenteral formulation. We have further discovered that the aqueous solubility of the drug is low and can be enhanced by increased pH, cosolvents and, by formation of inclusion complex.

Related compounds include, but are not limited to (E)-4-fluorostyryl-4-chlorobenzylsulfone; (E)4-chlorostyryl-4-chlorobenzylsulfone; (E)-2-chloro-4-fluorostyryl-4-chlorobenzylsulfone; (E)-4-carboxystyryl-4-chlorobenzyl sulfone; (E)-4-fluorostyryl-2,4-dichlorobenzylsulfone; (E)-4-fluorostyryl-4-bromobenzylsulfone; (E)-4-chlorostyryl-4-bromobenzylsulfone; (E)-4-bromostyryl-4-chlorobenzylsulfone; (E)-4-fluorostyryl-4-trifluoromethylbenzylsulfone; (E)-4-fluorostyryl-3,4-dichlorobenzylsulfone; (E)-4-fluorostyryl-4-cyanobenzylsulfone; (E)-2,4-dichloro-4-chlorobenzylsulfone; and (E)-4-chlorostyryl-2,4-dichlorobenzylsulfone

Related compounds include, but are not limited to (Z)-4-chlorostyryl-4-chlorobenzylsulfone; (Z)-4-chlorostyryl-4-fluorobenzylsulfone; (Z)-4-fluorostyryl-4-chlorobenzylsulfone; (Z)-4-bromostyryl-4-chlorobenzylsulfone; and (Z)-4-bromostyryl-4-fluorobenzylsulfone.

All related compounds disclosed, contemplated, or exemplified in U.S. Patent No.6,656,973, including but not limited to synthesis examples 1-219, are herein incorporated by reference.

EXAMPLES

## EXAMPLE I

### Preparation of ON.1210.Na salt

4-Chlorobenzyl-4-carboxystyryl sulfone (ON 01210) (49 g; 0.145 mol) was taken in a one-liter conical flask and 500 ml of distilled water was added. Sodium hydroxide solution (16 ml : 10 M stock) (0.150 mol.) was added to the conical flask. The contents of the flask were then boiled with stirring till ON.01210 was completely dissolved. The solution was then cooled to room temperature and shining crystals separated were filtered through a fluted filter paper. The crystalline material was dried under vacuum to yield (48 g) (92% yield) of pure ON.1210.Na.

## EXAMPLE II

### Radioprotective Effects of α,β-Unsaturated Arylsulfones on Cultured Normal Cells

The radioprotective effects of the compounds in Table 28 below on cultured normal cells were evaluated as follows.

HFL-1 cells, which are normal diploid lung fibroblasts, were plated into 24 well dishes at a cell density of 3000 cells per 10 $mm^2$ in DMEM completed with 10% fetal bovine serum and antibiotics. The test compounds listed in Table 28 were added to the cells 24 hours later in select concentrations from 2.5 to 20 micromolar, inclusive, using DMSO as a solvent. Control cells were treated with DMSO alone. The cells were exposed to the test compound or DMSO for 24 hrs. The cells were then irradiated with either 10 Gy (gray) or 15 Gy of ionizing radiation (IR) using a J.L. Shepherd Mark I, Model 30-1 Irradiator equipped with $^{137}$cesium as a source.

After irradiation, the medium on the test and control cells was removed and replaced with fresh growth medium without the test compounds or DMSO. The irradiated cells were incubated for 96 hours and duplicate wells were trypsinized and replated onto 100 $mm^2$ tissue culture dishes. The replated cells were grown under normal conditions with one change of fresh medium for 3 weeks. The number of colonies from each 100 $mm^2$ culture dish, which represents the number of surviving cells, was determined by staining the dishes as described below.

To visualize and count the colonies derived from the clonal outgrowth of individual radioprotected cells, the medium was removed and the plates were washed one time with room temperature phosphate buffered saline. The cells were stained with a 1:10 diluted Modified Giemsa staining solution (Sigma) for 20 minutes. The stain was removed, and the plates were washed with tap water. The plates were air dried, the number of colonies from each plate were counted and the average from duplicate plates was determined.

The results are presented in Table 28. A "+" indicates radioprotective activity of between 2- and 4.5-fold at the concentrations tested. Fold protection was determined by dividing the average number of colonies from the test plates by the average number of colonies on the control plates.

**Table 28: Radioprotection by α,β-Unsaturated Arylsulfones**

| Compound Number | Chemical Name | Activity |
|---|---|---|
| 1 | (E)-4-Fluorostyryl-4-chlorobenzylsulfone | + |
| 2 | (E)-2,4,6-Trimethoxystyryl-4-methoxybenzylsulfone | + |
| 3 | (E)-2-Methoxystyryl-4-nitrobenzylsulfone | - |
| 4 | (E)-2,3,4,5,6-Pentafluorostyryl-4-methoxybenzylsulfone | - |
| 5 | (E)-4-Fluorostyryl-4-trifluoromethylbenzylsulfone | + |

(continued)

| Compound Number | Chemical Name | Activity |
|---|---|---|
| 6 | (E)-4-Fluorostyryl-4-cyanobenzylsulfone | + |
| 7 | (Z)-4-Fluorostyryl-4-chlorobenzylsulfone | + |
| 8 | (E)-3-Furanethenyl-2,4-dichlorobenzylsulfone | + |
| 9 | (E)-4-Pyridylethenyl-4-chlorobenzylsulfone | - |
| 10 | (E)-4-Fluorostyryl-4-chlorophenylsulfone | + |
| 11 | (Z)-Styryl-(E)-2-methoxy-4-ethoxystyrylsulfone | + |
| 12 | (E)-4-Hydroxystyryl-4-chlorobenzylsulfone | - |
| 13 | (E)-4-Carboxystyryl-4-chlorobenzylsulfone | + |

## EXAMPLE III

### Protection of Mice from Radiation Toxicity by Pre-Treatment With (E)-4-Carboxystyryl-4-Chlorobenzylsulfone

C57 black mice age 10-12 weeks (Taconic) were divided into two treatment groups of 10 mice each. One group, designated the "200x2" group, received intraperitoneal injections of 200 micrograms (E)-4-carboxystyryl-4-chlorobenzylsulfone dissolved in DMSO (a 10 mg/Kg dose, based on 20 g mice) 18 and 6 hours before irradiation with 8 Gy gamma radiation. The other group, designated "500x2," received intraperitoneal injections of 500 micrograms (E)-4-carboxystyryl-4-chlorobenzylsulfone dissolved in DMSO (a 25 mg/Kg dose, based on 20 g mice) 18 and 6 hours before irradiation with 8 Gy gamma radiation. A control group of 16 animals received 8 Gy gamma radiation alone. Mortality of control and experimental groups was assessed for 40 days after irradiation, and the results are shown in FIG.5.

By day 20 post-irradiation, the control mice exhibited a maximum mortality rate of 80%; the 8 Gy dose of gamma radiation is thus considered an $LD_{80}$ dose. By contrast, only about 50% of the "200x2" group and about 30% of the "500x2" mice were dead at day 20 after receiving the $LD_{80}$ radiation dose. By day 40, a maximum mortality rate of approximately 60% was reached in the "200x2" group, and a maximum mortality rate of approximately 50% was reached in the "500x2" group. These data show that radiation toxicity in mice is significantly reduced by pre-treatment with (E)-4-carboxystyryl-4-chlorobenzylsulfone.

## EXAMPLE IV

### Radioprotective Effect of (E)-4-Carboxystyryl-4-chlorobenzylsulfone in Mice When Given After Radiation Exposure

C57 B6/J mice age 10-12 weeks (Taconic) were divided into two treatment groups of 10 and 9 mice, respectively. One group, designated the "200x2" group, received intraperitoneal injections of 200 micrograms (E)-4-carboxystyryl-4-chlorobenzylsulfone dissolved in DMSO (a 10 mg/Kg dose, assuming 20 g mice) 18 and 6 hours before irradiation with 8 Gy gamma radiation. The other group, designated "200 Post," received an intraperitoneal injection of 200 micrograms (E)-4-carboxystyryl-4-chlorobenzylsulfone dissolved in DMSO (a 10 mg/Kg dose, based on 20 g mice) 15 minutes after irradiation with 8 Gy gamma radiation. A control group of 16 animals received 8 Gy gamma radiation alone. Mortality of control and experimental groups was assessed for 40 days after irradiation, and the results are shown in FIG.6.

FIG.6 shows that treatment of mice with (E)-4-carboxystyryl-4-chlorobenzylsulfone after irradiation resulted in significant delay in radiation-induced mortality as compared with the control animals. While the radioprotection afforded by post-irradiation treatment is not as great as seen with pre-irradiation treatment, (E)-4-carboxystyryl-4-chlorobenzylsulfone is nonetheless effective in mitigating the effects of radiation toxicity after the subject has received the radiation dose.

Although the invention has been described in connection with specific embodiments, it should be understood that the invention as claimed should not be unduly limited to these embodiments. Indeed, various modifications for carrying out the invention are obvious to those skilled in the art and are intended to be within the scope of the following claims.

## Claims

1. A pharmaceutical composition for use in reducing the toxic effects of ionizing radiation in a subject comprising between 20mg/ml to 60mg/ml of the sodium salt of (E)-4-carboxystyryl-4-chlorobenzylsulfone (ON.1210.Na) and at least one component selected from the group consisting of a) at least one chemically modified cyclodextrin selected from the group consisting of 2-hydroxypropyl-beta-cyclodextrin, 2-hydroxypropyl-gamma-cyclodextrin, and hydroxyethyl-beta-cyclodextrin in an amount between 20% and 60% w/v, b) a water soluble polymer selected from the group consisting of povidone in an amount between 0.5% and 20% w/v and PEG in an amount between 25% and 90% w/v, and c) DMA (N,N-dimethylacetamide) in an amount between 10% and 50% w/v, wherein the composition has a pH within the range of 7.5 to 9.2.

2. The composition of claim 1 which comprises between 30mg/ml to 50mg/ml of the compound ON.1210.Na and between 30% and 50% w/v 2-hydroxypropyl-beta-cyclodextrin.

3. The composition of claim 1 which comprises between 30mg/ml to 50mg/ml of the compound ON.1210.Na and between 15% and 40% w/v DMA.

4. The composition of claim 1 which comprises between 30mg/ml to 50mg/ml of the compound ON.1210.Na and at least 50% PEG w/v.

5. The composition of claim 1 which comprises between 30mg/ml to 50mg/ml of the compound ON.1210.Na and between 1% and 10% povidone w/v.

6. The composition of claim 1, wherein the water soluble polymer is 1% w/v povidone K90 in water.

## Patentansprüche

1. Pharmazeutische Zusammensetzung zur Verwendung bei der Verringerung der toxischen Wirkungen von ionisierender Strahlung bei einem Patienten, umfassend zwischen 20 mg/ml bis 60 mg/ml des Natriumsalzes von (E)-4-Carboxystyryl-4-chlorbenzylsulfon (ON.1210.Na) und mindestens eine Komponente aus der Gruppe bestehend aus a) mindestens einem chemisch modifizierten Cyclodextrin aus der Gruppe bestehend aus 2-Hydroxypropyl-beta-cyclodextrin, 2-Hydroxypropyl-gamma-cyclodextrin und Hydroxyethyl-beta-cyclodextrin in einer Menge zwischen 20 und 60% w/v, b) einem wasserlöslichen Polymer aus der Gruppe bestehend aus Povidon in einer Menge zwischen 0,5 und 20% w/v und PEG in einer Menge zwischen 25 und 90% w/v und c) DMA (N,N-Dimethylacetamid) in einer Menge zwischen 10 und 50% w/v, wobei die Zusammensetzung einen pH-Wert im Bereich von 7,5 bis 9,2 aufweist.

2. Zusammensetzung nach Anspruch 1, die zwischen 30 mg/ml bis 50 mg/ml der Verbindung ON:1210.Na und zwischen 30 und 50% w/v 2-Hydroxypropyl-beta-cyclodextrin umfasst.

3. Zusammensetzung nach Anspruch 1, die zwischen 30 mg/ml bis 50 mg/ml der Verbindung ON:1210.Na und zwischen 15 und 40% w/v DMA umfasst.

4. Zusammensetzung nach Anspruch 1, die zwischen 30 mg/ml bis 50 mg/ml der Verbindung ON:1210.Na und mindestens 50% w/v PEG umfasst.

5. Zusammensetzung nach Anspruch 1, die zwischen 30 mg/ml bis 50 mg/ml der Verbindung ON:1210.Na und zwischen 1 und 10% w/v Povidon umfasst.

6. Zusammensetzung nach Anspruch 1, wobei es sich bei dem wasserlöslichen Polymer um 1% w/v Povidon K90 in Wasser handelt.

## Revendications

1. Composition pharmaceutique destinée à être utilisée pour la réduction des effets toxiques de radiations ionisantes chez un sujet, comprenant entre 20 mg/ml et 60 mg/ml du sel sodique de (E)-4-carboxystyryl-4-chlorobenzylsulfone

(ON.1210.Na) et au moins un composant choisi dans le groupe constitué par a) au moins une cyclodextrine chimiquement modifiée, choisie dans le groupe constitué par la 2-hydroxypropyl-bêta-cyclodextrine, la 2-hydroxypropyl-gamma-cyclodextrine, et l'hydroxyéthyl-bêta-cyclodextrine dans une quantité comprise entre 20 % et 60 % p/v, b) un polymère hydrosoluble choisi dans le groupe constitué par la povidone dans une quantité comprise entre 0,5 % et 20 % p/v et le PEG dans une quantité comprise entre 25 % et 90 % p/v, et c) le DMA (N,N-diméthylacétamide) dans une quantité comprise entre 10 % et 50% p/v, la composition ayant un pH dans la gamme de 7,5 à 9,2.

**2.** Composition selon la revendication 1, **caractérisée en ce qu'**elle comprend entre 30 mg/ml et 50 mg/ml du composé ON.1210.Na et entre 30 % et 50 % p/v de 2-hydroxypropyl-bêta-cyclodextrine.

**3.** Composition selon la revendication 1, **caractérisée en ce qu'**elle comprend entre 30 mg/ml et 50 mg/ml du composé ON.1210.Na et entre 15 % et 40 % p/v de DMA.

**4.** Composition selon la revendication 1, **caractérisée en ce qu'**elle comprend entre 30 mg/ml et 50 mg/ml du composé ON.1210.Na et au moins 50 % p/v de PEG.

**5.** Composition selon la revendication 1, **caractérisée en ce qu'**elle comprend entre 30 mg/ml et 50 mg/ml du composé ON.1210.Na et entre 1 % et 10% p/v de povidone.

**6.** Composition selon la revendication 1, **caractérisée en ce que** le polymère hydrosoluble est la povidone K90 à 1 % p/v dans l'eau.

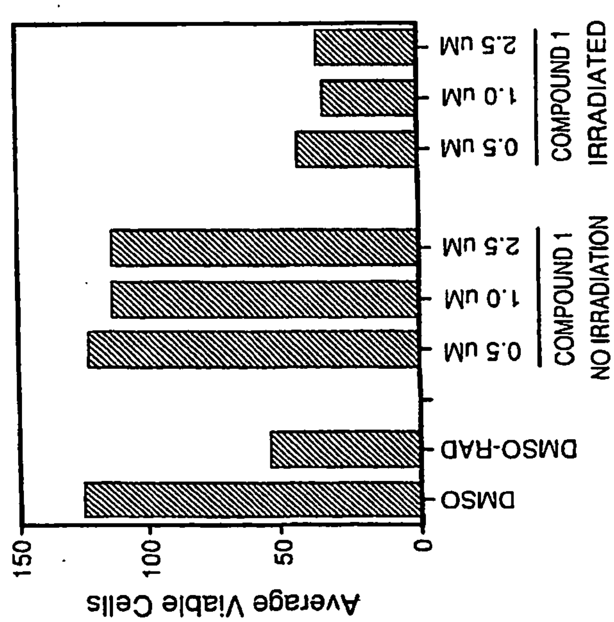
Average Viable Cells
150
100
50
0
DMSO
DMSO-RAD
0.5 uM
1.0 uM
2.5 uM
COMPOUND 1
NO IRRADIATION
0.5 uM
1.0 uM
2.5 uM
COMPOUND 1
IRRADIATED

FIG. 1A

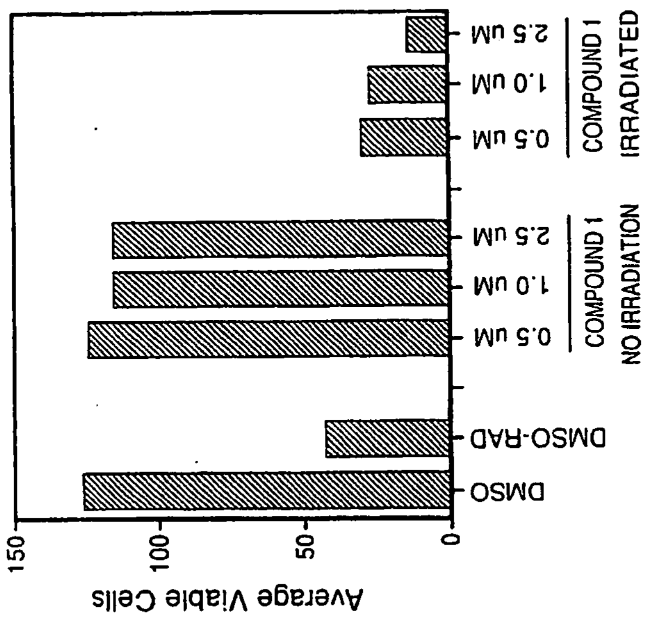
Average Viable Cells
150
100
50
0
DMSO
DMSO-RAD
0.5 uM
1.0 uM
2.5 uM
COMPOUND 1
NO IRRADIATION
0.5 uM
1.0 uM
2.5 uM
COMPOUND 1
IRRADIATED

FIG. 1B

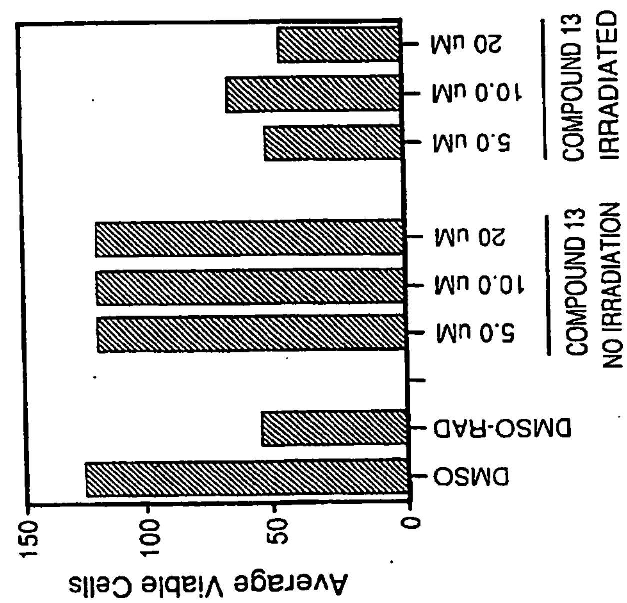
Average Viable Cells
150
100
50
0
DMSO
DMSO-RAD
5.0 uM
10.0 uM
20 uM
COMPOUND 13
NO IRRADIATION
5.0 uM
10.0 uM
20 uM
COMPOUND 13
IRRADIATED

FIG. 2A

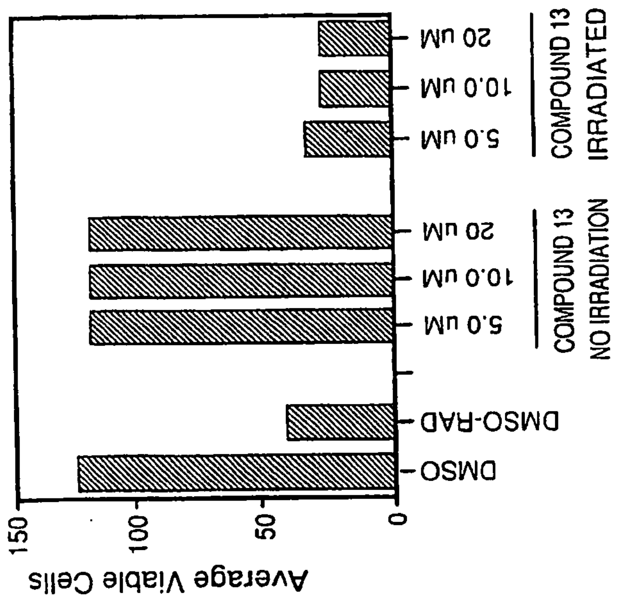
Average Viable Cells
150
100
50
0
DMSO
DMSO-RAD
5.0 uM
10.0 uM
20 uM
COMPOUND 13
NO IRRADIATION
5.0 uM
10.0 uM
20 uM
COMPOUND 13
IRRADIATED

FIG. 2B

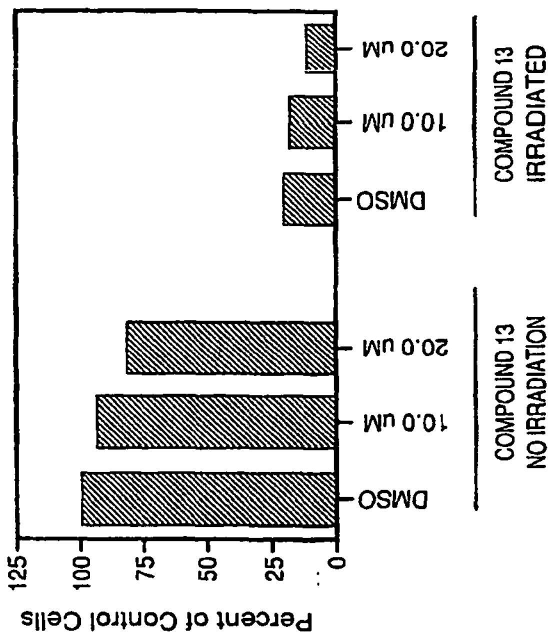
Percent of Control Cells
125
100
75
50
25
0
DMSO
10.0 uM
20.0 uM
DMSO
10.0 uM
20.0 uM
COMPOUND 13
NO IRRADIATION
COMPOUND 13
IRRADIATED

FIG. 3B

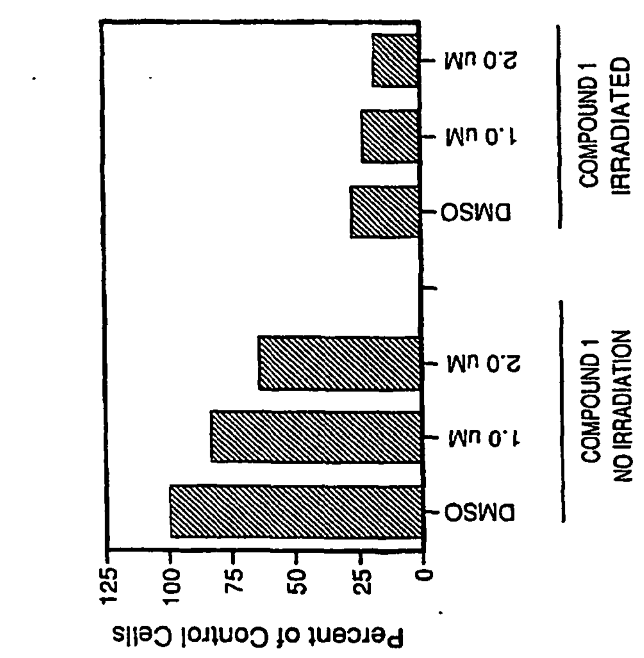
Percent of Control Cells
125
100
75
50
25
0
DMSO
1.0 uM
2.0 uM
DMSO
1.0 uM
2.0 uM
COMPOUND 1
NO IRRADIATION
COMPOUND 1
IRRADIATED

FIG. 3A

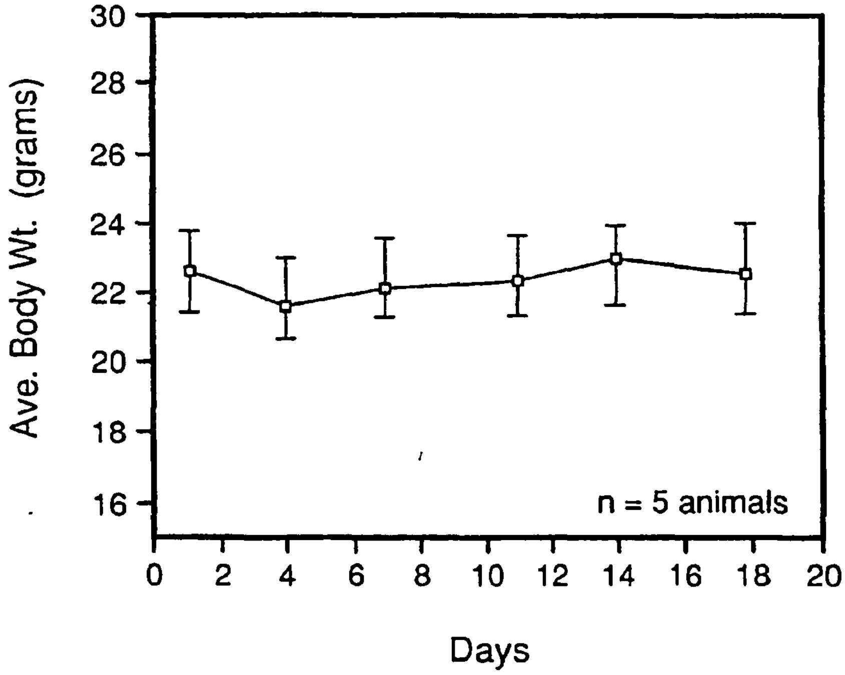
Ave. Body Wt. (grams)
30
28
26
24
22
20
18
16
n = 5 animals
0
2
4
6
8
10
12
14
16
18
20
Days

FIG. 4

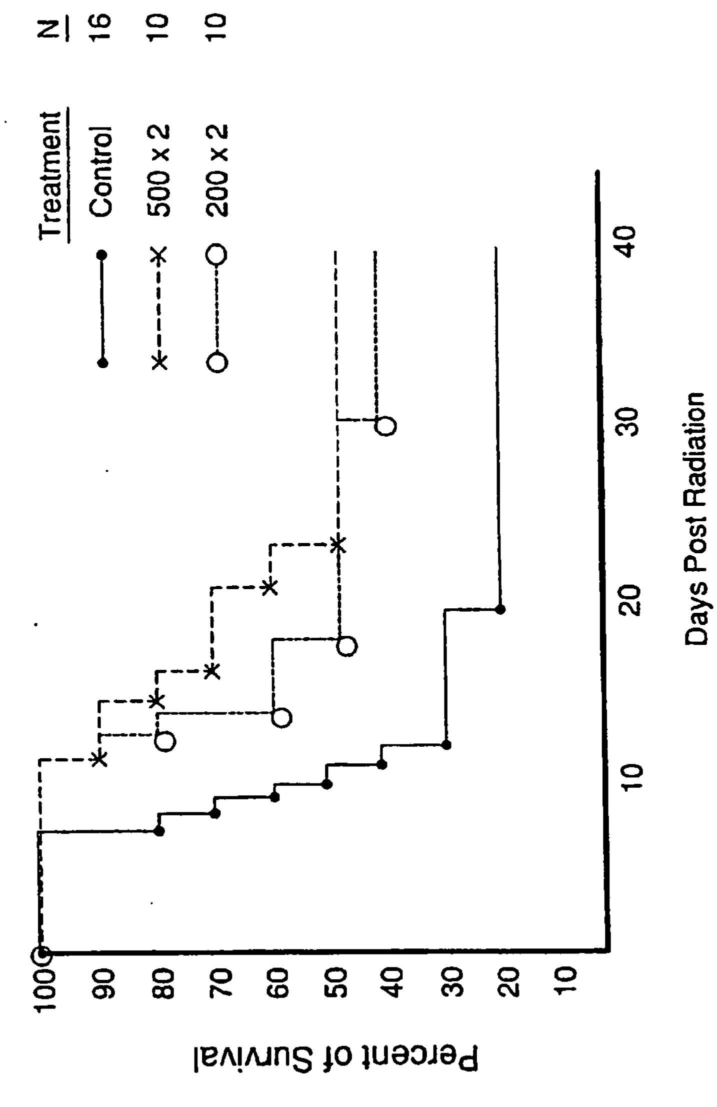
Treatment
Control
500 x 2
200 x 2
N
16
10
10
Percent of Survival
100
90
80
70
60
50
40
30
20
10
10
20
30
40
Days Post Radiation

FIG. 5

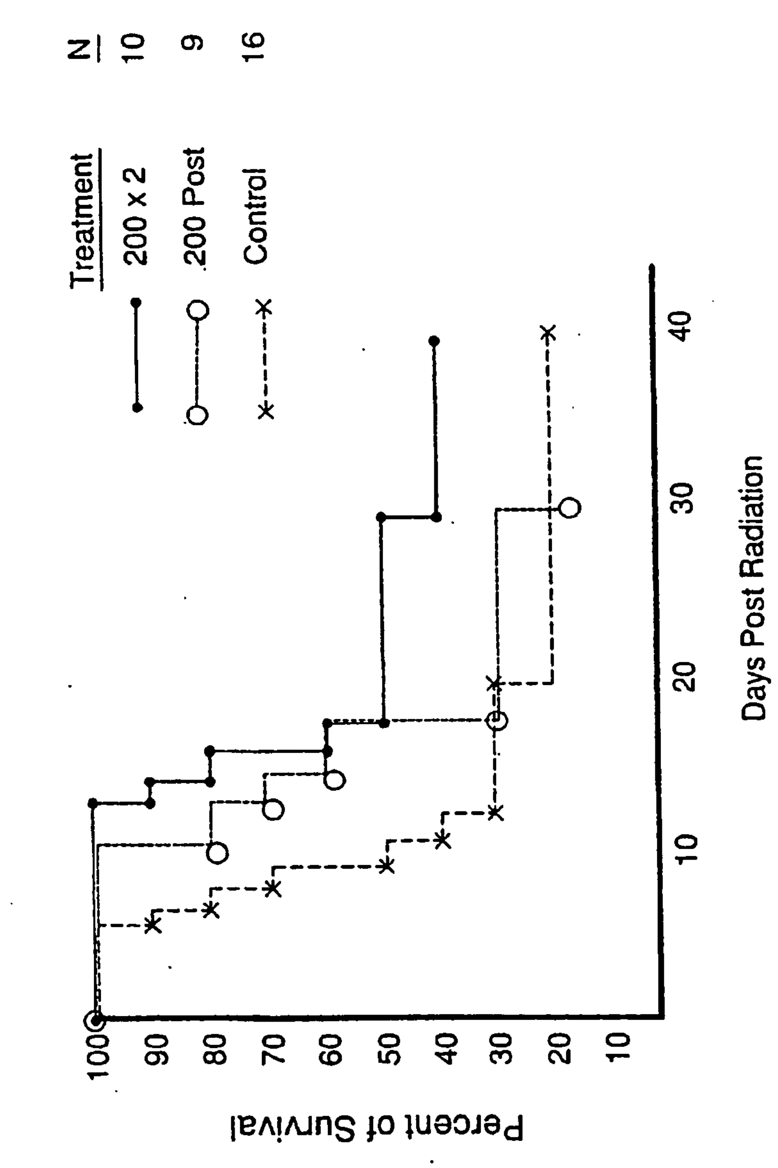
Percent of Survival
100
90
80
70
60
50
40
30
20
10
Days Post Radiation
10
20
30
40
Treatment
N
200 x 2
10
200 Post
9
Control
16

FIG. 6

**(E)-4-Carboxystyryl-4-Chlorobenzyl sulfone**

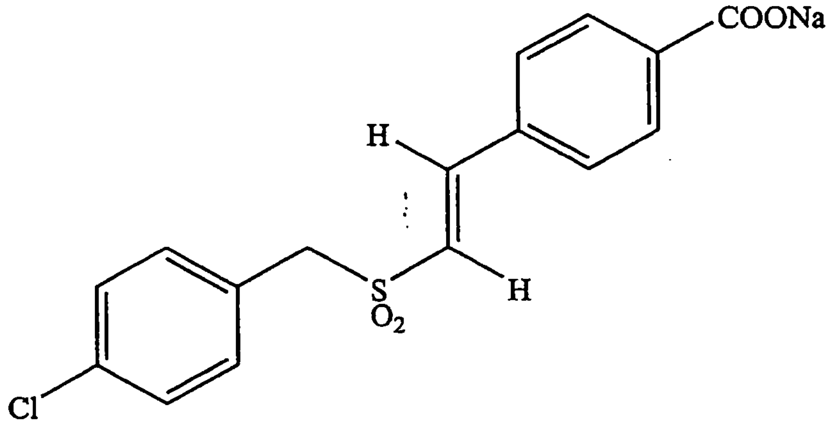

**ON 01210.Na**

FIG. 7

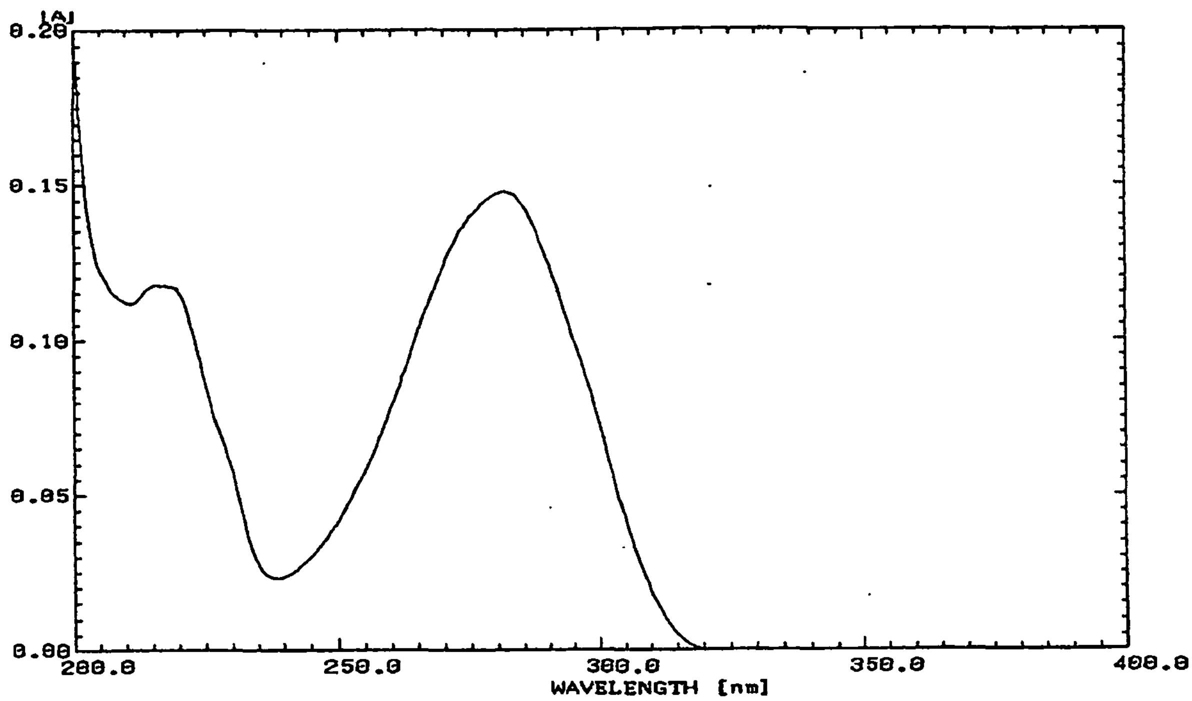


FIG. 8

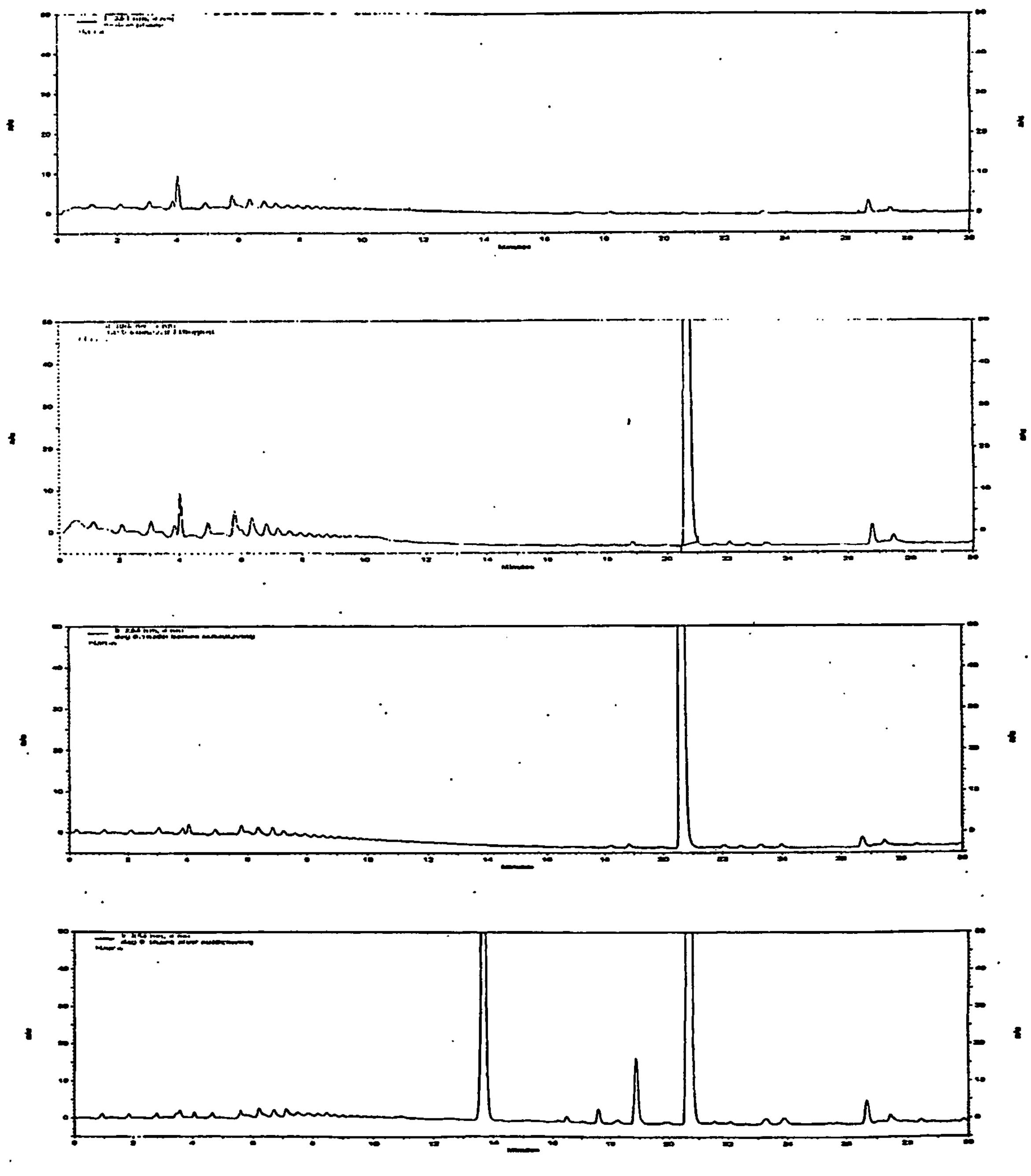

FIG. 9

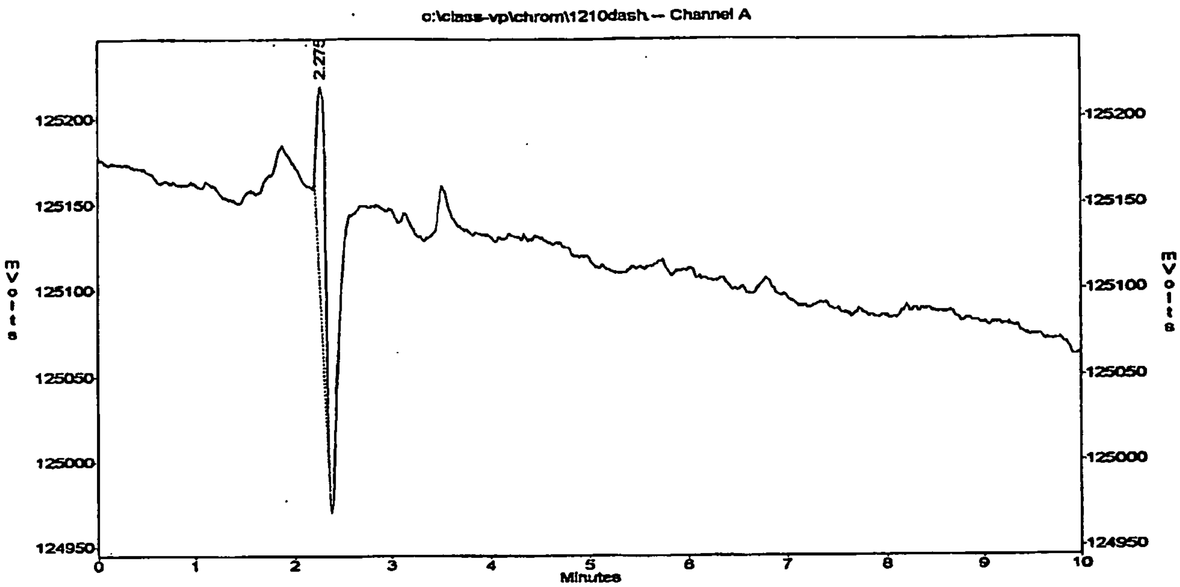


Channel A Results

FIG. 10A

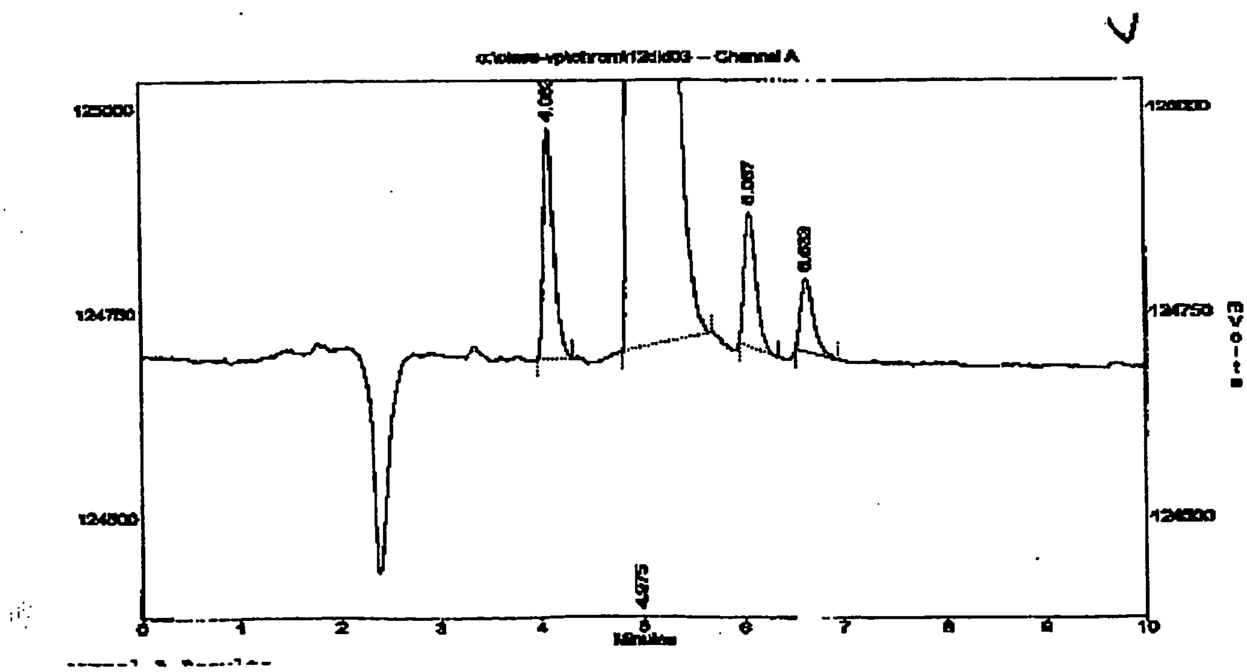


FIG. 10B

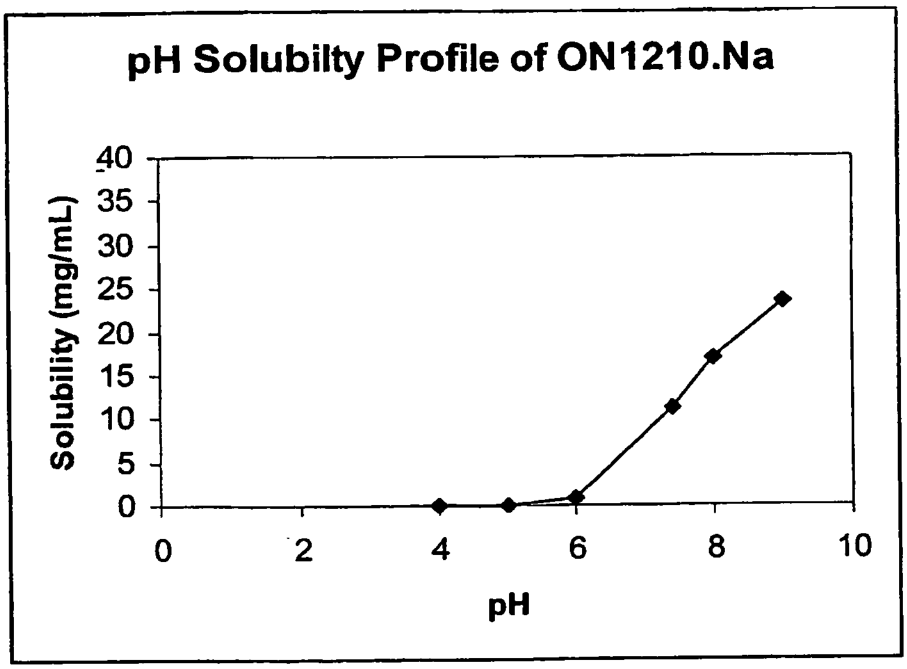
pH Solubilty Profile of ON1210.Na
Solubility (mg/mL)
40
35
30
25
20
15
10
5
0
0
2
4
6
8
10
pH

FIG. 11

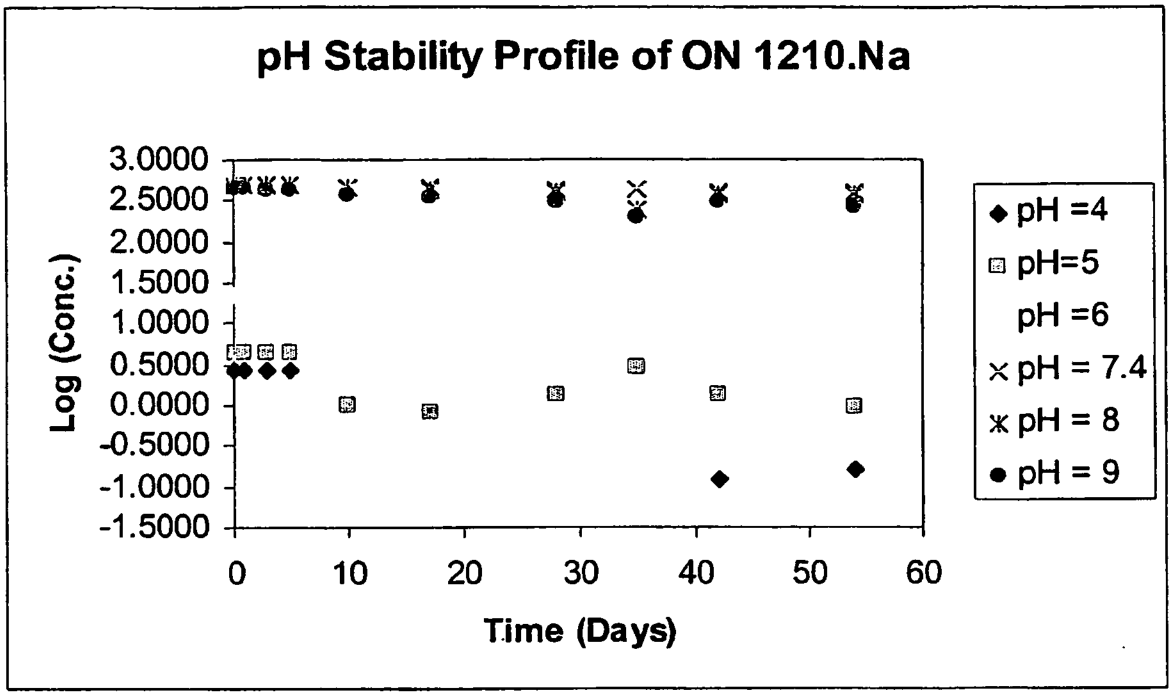
pH Stability Profile of ON 1210.Na
3.0000
2.5000
2.0000
1.5000
1.0000
0.5000
0.0000
-0.5000
-1.0000
-1.5000
Log (Conc.)
0
10
20
30
40
50
60
Time (Days)
pH =4
pH=5
pH =6
pH = 7.4
pH = 8
pH = 9

FIG. 12

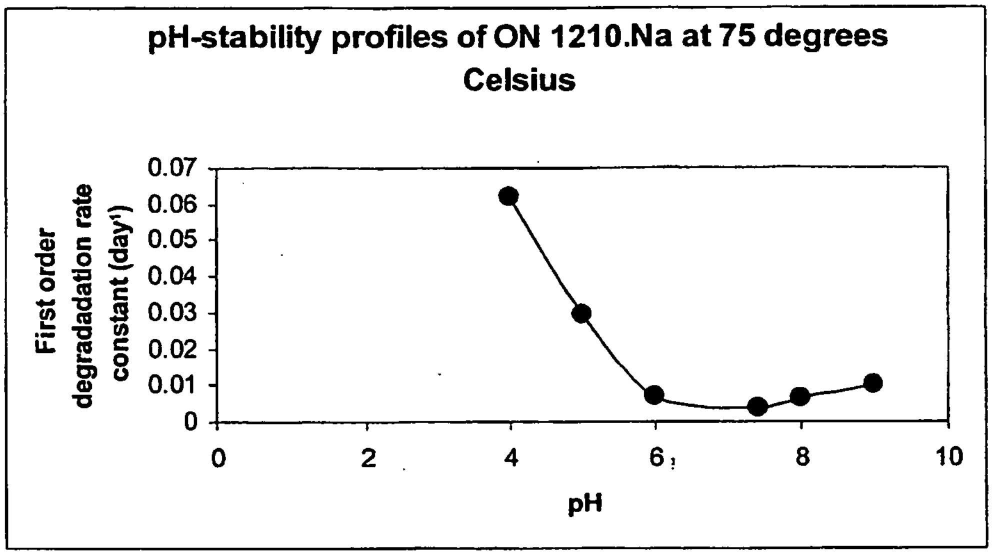
pH-stability profiles of ON 1210.Na at 75 degrees Celsius
First order degradadation rate constant (day⁻¹)
0.07
0.06
0.05
0.04
0.03
0.02
0.01
0
0
2
4
6
8
10
pH

FIG. 13

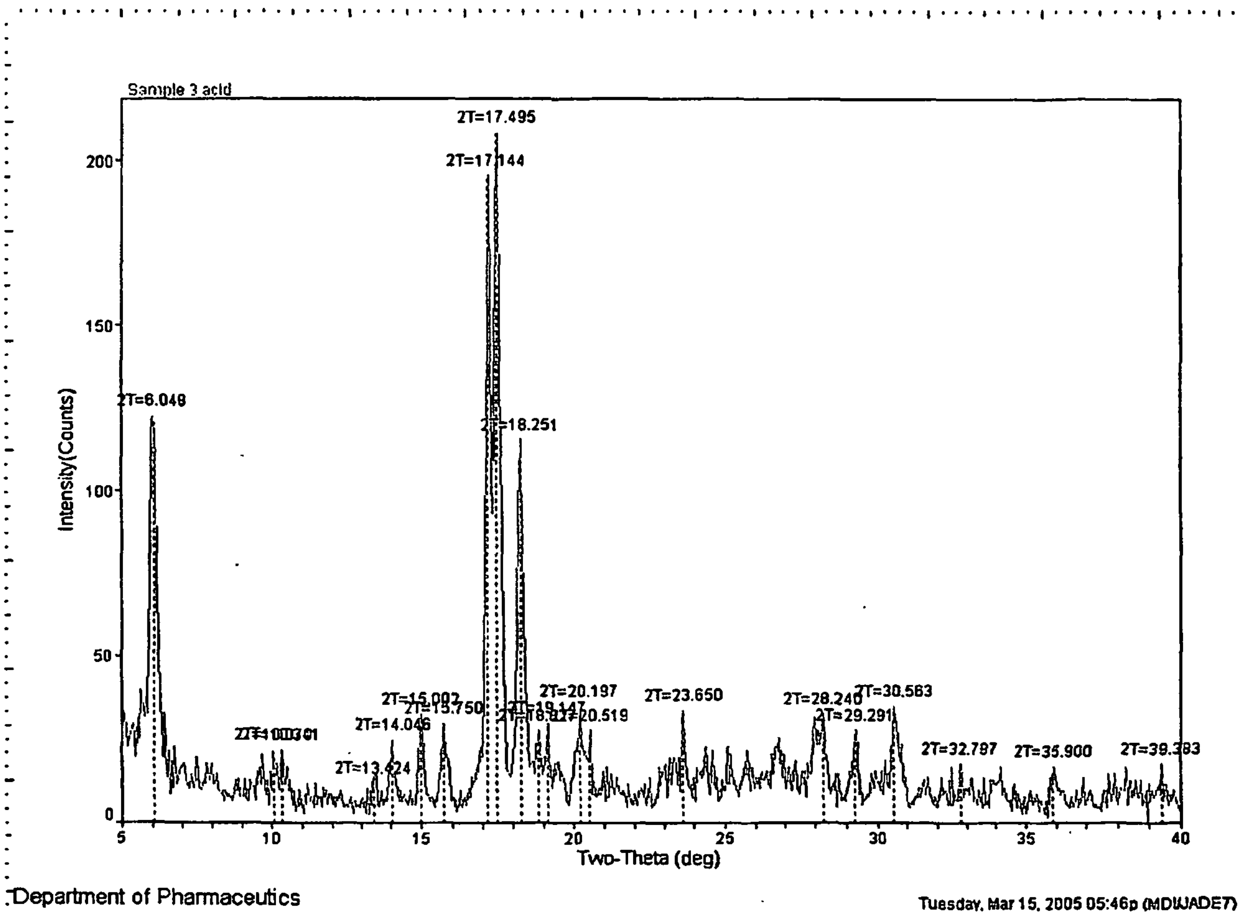
Sample 3 acid
2T=17.495
2T=17.144
2T=6.048
2T=18.251
2T=20.197
2T=23.650
2T=30.563
2T=28.240
2T=29.291
2T=14.046
2T=13.424
2T=20.519
2T=32.797
2T=35.900
Intensity(Counts)
Two-Theta (deg)
Department of Pharmaceutics
Tuesday, Mar 15, 2005 05:46p (MDI/JADE7)

FIG. 14

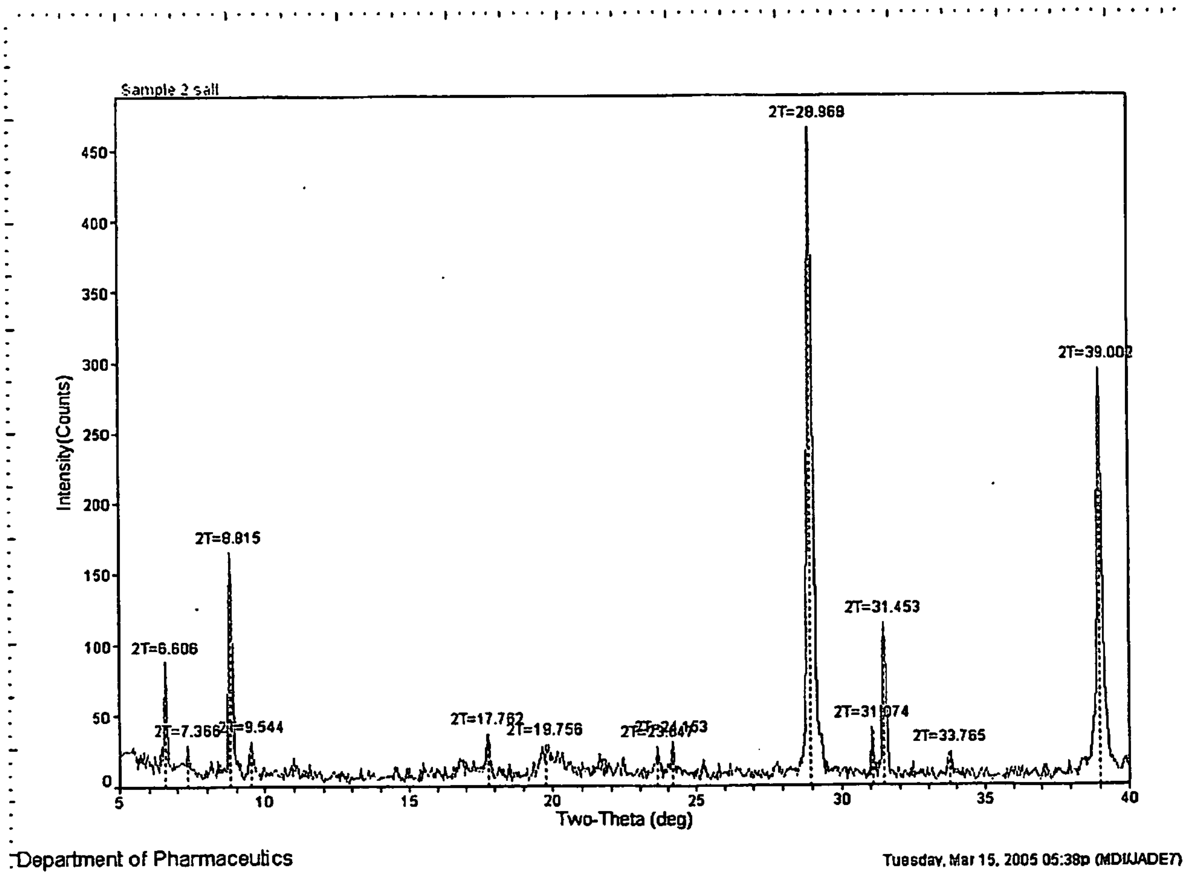
Sample 2 salt
Intensity(Counts)
Two-Theta (deg)
2T=28.969
2T=39.002
2T=8.815
2T=31.453
2T=6.606
2T=7.366
2T=9.544
2T=17.762
2T=19.756
2T=23.647
2T=24.163
2T=31.074
2T=33.765
Department of Pharmaceutics
Tuesday, Mar 15, 2005 05:38p (MDI/JADE7)

FIG. 15

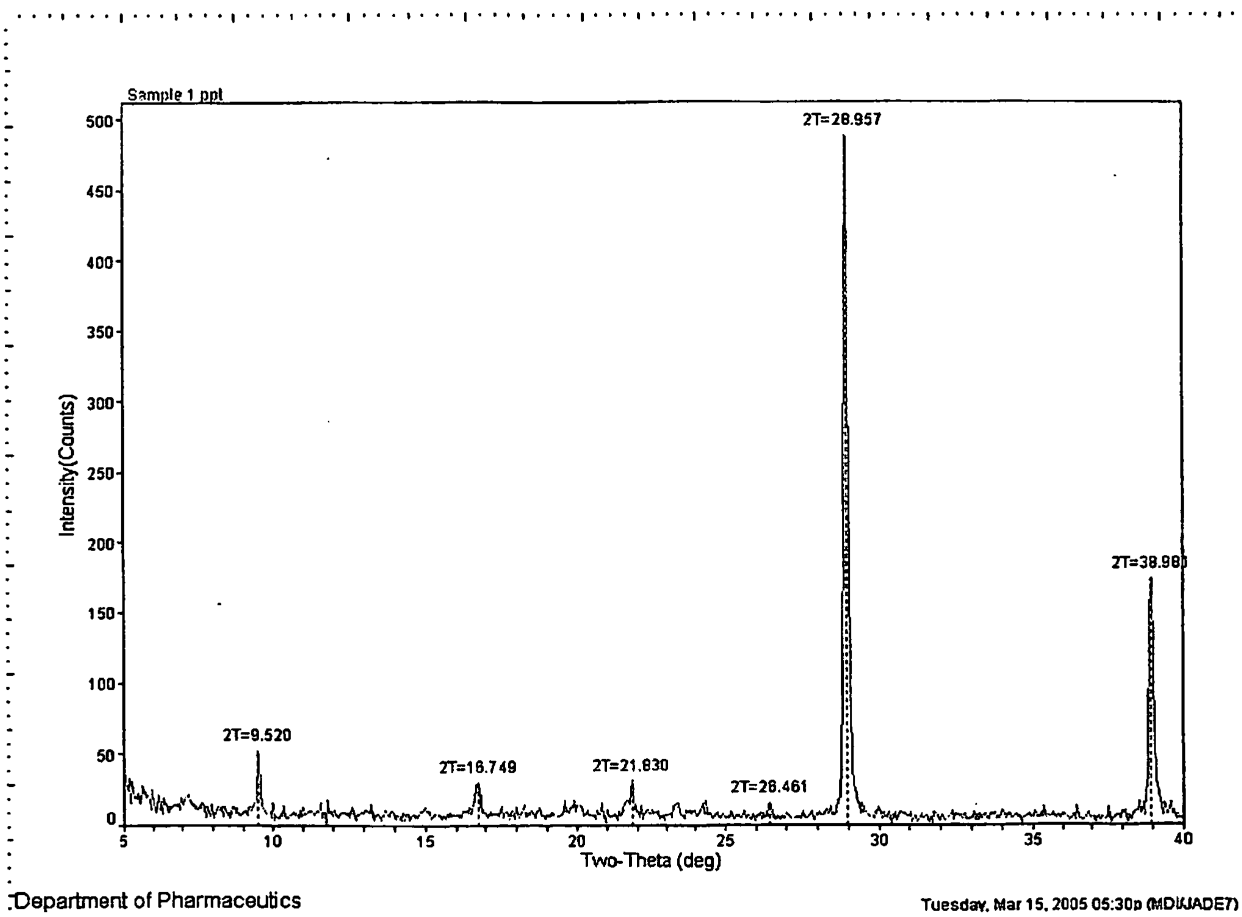
Sample 1 ppt
500
450
400
350
300
250
200
150
100
50
0
Intensity(Counts)
2T=9.520
2T=16.749
2T=21.830
2T=26.461
2T=28.957
2T=38.98
5
10
15
20
25
30
35
40
Two-Theta (deg)
Department of Pharmaceutics
Tuesday, Mar 15, 2005 05:30p (MDI/JADE7)

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20030060505 A
- US 6656973 B2
- US 6656973 B
- US 6667346 B
- US 4727064 A, Pitha, Josef
- US 4764604 A, Muller, B.W.


### Non-patent literature cited in the description


- **Neta et al.** *Semin. Radiat. Oncol.,* 1996, vol. 6, 306-320
- **Maisin, J.R. ; Bacq ; Alexander.** Chemical radioprotection: past, present, and future prospects. *Int J. Radiat Biol.,* 1998, vol. 73, 443-50
- **Alfieri et al.** Proceedings of the 46th Annual ASTRO Meeting. *International Journal of Radiation Oncology Biology Physics,* 2004, vol. 60 (1), S367-S368
- **Reddy et al.** *Acta. Chim. Hung.,* 1984, vol. 115, 269-71
- **Reddy et al.** *Sulfur Letters,* 1991, vol. 13, 83-90
- **Reddy et al.** *Synthesis,* 1984, vol. 4, 322-23
- **Reddy et al.** *Sulfur Letters,* 1987, vol. 7, 43-48
- Comprehensive Supramolecular Chemistry. Elsevier Science Inc, vol. 3-11, 10591-5153
- **Yoshida, A. et al.** Pharmaceutical Evaluation Of Hydroxy Alkyl Ethers Of B-Cyclodextrins. *Int. Pharm,* 1988, vol. 46, 217
- **Muller, B. W.** Hydroxypropyl-B-Cyclodextrin Derivatives: Influence Of Average Degree Of Substitution On Complexing Ability And Surface Activity. *J. Pharm Sci.,* June 1986, vol. 75 (6
- **Irie, T. et al.** Amorphous Water- Soluble Cyclodextrin Derivatives: 2-hydroxyethyl, 3-hydroxypropyl, 2-hyroxyisobutyl, and carboxamidomethyl derivatives of B-cyclodextrin. *Pharm Res.,* 1988, 713
- **Pitha, Josef et al.** Drug Solubilizers To Aid Pharmacologists: Amorphous Cyclodextrin Derivatives. *Life Sciences.,* 1988, vol. 43 (6), 493-502